# EUROPEAN PATENT APPLICATION

(11) **EP 3 466 413 A1**
(43) Date of publication of application: **10.04.2019**
(21) Application number: 17195499.3
(22) Date of filing: 09.10.2017
(51) Int. Cl.: A61K 9/20, A61K 47/12, A61K 31/165

(54) **PHARMACEUTICAL COMPOSITION CONTAINING AGOMELATINE AND PROCESS FOR THE PREPARATION THEREOF**

(71) Applicant: KRKA, d.d., Novo mesto, 8501 Novo mesto (SI)
(72) Inventor: Vrbinc, Miha, 8000 Novo mesto (SI); Pavlin, Darja, 8351 Straza pri Novem mesto (SI); Meznar, Klavdija, 8000 Novo mesto (SI); Mateovic-Rojnik, Tatjana, 1000 Lubljana (SI); Vrecer, Franc, 8351 Straza pri Novem mestu (SI)
(74) Representative: Hoefer & Partner Patentanwälte mbB

(57) **Abstract**

The present invention relates to a pharmaceutical composition comprising agomelatine, in particular agomelatine in crystalline form, especially agomelatine in in polymorphic form X or I, and a pH modifier and optionally polymeric material, as well as to a process for the preparation thereof.

## Description

### TECHNICAL FIELD OF THE INVENTION

The present invention relates to a (stable) pharmaceutical composition comprising agomelatine, in particular agomelatine in crystalline form, especially agomelatine in polymorphic form X or I, and a pH modifier and optionally polymeric material, as well as to a process for the preparation thereof. The pH modifier, and optionally polymeric material, can enhance the physicochemical stability of agomelatine in the pharmaceutical composition, in particular in the finished dosage form, and enable the required dissolution and bioavailability of the drug.

### BACKGROUND OF THE INVENTION

Agomelatine provides an advantageous concept for antidepressant treatment. The agomelatine molecule possesses a pharmacological mechanism of action, which combines its melatonin MT1 and MT2 agonist properties with a serotonin 5-HT_{2C} antagonist effect. The 5-HT_{2C} receptors are considered as a relevant target with regard to antidepressant therapy, as several currently used antidepressant drugs are endowed with 5-HT_{2C} receptor antagonist properties (e.g. mianserin and mirtazapine).

Agomelatine is a melatonin receptor agonist and a serotonin 5-HT_{2C} receptor antagonist used for the treatment of major depressive disorder. Agomelatine' s chemical name is N[2-(7-methoxy-1-naphthyl)ethyl] acetamide, and its chemical structure is presented by the following Formula (I).

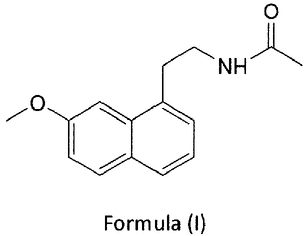

Agomelatine is a white to almost white crystalline powder that is non-hygroscopic; while it is practically insoluble in water, it is very slightly soluble in aqueous buffers, and freely soluble in organic solvents such as methanol and dichloromethane. The solubility of the drug in these two before-mentioned solvents is approximately 500 and 80 mg/ml, respectively.

Agomelatine is a highly permeable drug, but it is only very slightly soluble in aqueous solution over the physiological pH range. This poor solubility of agomelatine in aqueous media poses a challenge to the pharmaceutical formulation scientist as an enhancement in the dissolution rate and bypass of first-pass metabolism should be considered during formulation development in order to have maximum therapeutic efficacy.

Furthermore, modifications in the solid state, conversion from one polymorph to another, solvation/hydration, or amorphization have to be considered during drug development of poorly-soluble drugs.

Generally, metastable forms are more soluble than the corresponding stable polymorphic forms, but they can transform to the more thermodynamically stable form in a relatively short time. Thus, it is necessary to monitor the polymorphic transformation during formulation, manufacturing, and storage of dosage forms to ensure reproducible bioavailability after administration.
In addition, the change of the polymorphic form may cause clinical failures. It is therefore crucial that any transformation of the polymorphic forms during shelf life is prevented or minimized.

Agomelatine is an active pharmaceutical ingredient that exhibits polymorphism. EP-B-0 447 285 discloses a process for the preparation of agomelatine, wherein agomelatine obtained by this process is in crystalline form I. However, crystalline Form I may convert into more stable crystalline Form II when subjected to elevated temperatures or during formulation processes. Furthermore, several polymorphs of agomelatine are known from the literature such as crystalline Form V disclosed in EP-A-1 752 444, Crystalline Form V disclosed in EP-B-1 752 443, crystalline Form VI disclosed in EP-A-2 058 296, crystalline Form VII disclosed in EP-A- 2690087, and crystalline form X disclosed in EP- B- 2 474 522. Asian Journal of Pharmaceutical Sciences 8(2013) 181-190 describes crystalline Form I, II and III of agomelatine.

Solubility is an essential property of drugs, because they must dissolve in order to be absorbed through membranes and reach the site of action. Consequently, solubility is one of the most critical and important parameters influencing drug bioavailability, that is, the ability of a drug to be available in an appropriate concentration at the site of action, independently of the pharmaceutical dosage form and route of administration.

Various methods have been suggested for the industrial preparation of oral dosage forms comprising agomelatine or a derivative thereof, as an active ingredient. However, the prior art has encountered substantial difficulties in the production of a stable and bioavailable composition of agomelatine.

WO-A-2013/082302 discloses the formation of agomelatine - urea complex in order to improve the stability and shelf-life of the API and improve the dissolution profile or bioavailability of the finished product comprising said agomelatine - urea complex.

WO-A-2012/130837 discloses the use of hydrophilic polymers as surface stabilizer for the preparation of a composition comprising agomelatine in order to improve the stability of the active ingredient. Said composition is prepared by using melt extrusion process.

WO-A-2014/012571 discloses the formation of agomelatine - cyclodextrin complex and a composition comprising said complex.

As disclosed in Cryst. Growth Des. 2012, 12, 2226-2233, the solubility of polymorphic forms of agomelatine and agomelatine co-crystals differ in substantial extent.

WO 2014/194992 and WO 2014/194991 disclose pharmaceutical compositions comprising agomelatine co-crystal with citric acid.

WO-A-2014/095818 discloses the use of a copolymer of methacrylic acid and divinylbenzene for the preparation of a pharmaceutical composition of agomelatine, wherein agomelatine is present in the composition in stabilized amorphous form.

EP 3 087 977 A1 and EP 3 087 976 A1 disclose that polymorphic forms I and X have been stabilised in pharmaceutical composition by a protective agent.

Although each one of the documents above represents an attempt to improve pharmaceutical compositions comprising agomelatine, there still exists a need for further improving pharmaceutical compositions comprising agomelatine. In particular, there still exists a need for further improving stability and release rate of agomelatine in pharmaceutical compositions. Furthermore, there still exists a need for providing a less complicated production approach for pharmaceutical compositions comprising agomelatine.

### SUMMARY OF THE INVENTION

It is, therefore, an object of the present invention to provide a pharmaceutical composition, in particular for oral administration, which overcomes the deficiencies of the prior art compositions.

In particular, an object of the present invention is to provide a pharmaceutical composition for oral administration comprising agomelatine, with good or improved physicochemical characteristics and/or good or improved physicochemical stability of agomelatine, especially wherein its polymorphic form transformation during shelf life of the pharmaceutical composition of the present invention is prevented or minimized.
Another object of the present invention is to provide a pharmaceutical composition comprising agomelatine with advantageous dissolution and required bioavailability of the active ingredient agomelatine.
Moreover, it is another object of the present invention to provide a suitable process for the preparation of a pharmaceutical composition for oral administration comprising a therapeutically effective amount of agomelatine, which is cost effective and reproducible.

The present invention solves the before-mentioned problems by providing a pharmaceutical composition, in particular pharmaceutical composition for oral administration, comprising agomelatine (especially in a therapeutically effective amount), in particular agomelatine in crystalline form, and a pH modifying agent, and optionally comprising polymer. In particular, the present invention solves the problem of poor solubility by including into the pharmaceutical composition polymorphic form(s) with comparatively high solubility, such as metastable forms I and/or X, and provides a composition wherein conversion of polymorphic forms during pharmaceutical composition manufacture and shelf life is prevented or minimized.

The pharmaceutical compositions of the present invention comprising a pH modifier and optionally a polymer, provide pharmaceutical compositions with good physical and chemical stability during manufacturing and shelf life. In particular, the pharmaceutical compositions of the present invention prevent or reduce the conversion of polymorphic forms during the manufacturing of the pharmaceutical composition and during shelf life. The pharmaceutical compositions of the present invention can comprise agomelatine polymorphic forms with sufficient or high solubility, such as metastable forms I and X. In a pharmaceutical composition of the present invention, the presence of a pH modifying agent and optionally a polymer contributes to the provision of a pharmaceutical composition, which effectively stabilizes used used crystalline agomelatine polymorphic form, as well as contributes to advantageous dissolution properties and bioavailability. In particular, pharmaceutical compositions of the present invention comprising one or more agomelatine forms different from agomelatine polymorphic form II (such as e.g. forms I and/or X) showing a reduced tendency for conversion of these forms into agomelatine polymorphic form II, and/or a reduced tendency for the formation of agomelatine degradation products.
Furthermore, the present inventors provide a pharmaceutical composition for oral administration comprising a therapeutically effective amount of agomelatine, and an effective amount of pH modifier as a stabilizer in order to improve the physicochemical stability of the active ingredient in the finished dosage form.

According to another aspect, a process for the preparation of a pharmaceutical composition, in particular for oral administration, comprising a therapeutically effective amount of agomelatine, and an effective amount of pH modifier, is provided. Without wishing to be bound by theory, it is assumed that the pH modifier acts as stabilizer in the pharmaceutical composition in order to improve the physicochemical stability of agomelatine in the finished dosage form. Optionally, it is assumed that the pH modifier, especially the pH modifier and the polymer, act(s) as stabilizer(s) in the pharmaceutical composition in order to improve the physicochemical stability of agomelatine, especially physical stability of metastable polymorphs of agomelatine in the finished dosage form.

### DESCRIPTION OF THE INVENTION

The present invention relates to a pharmaceutical composition comprising agomelatine, in particular a therapeutically effective amount of agomelatine, especially a therapeutically effective amount of agomelatine in crystalline form, and at least one pH modifier, and optionally further comprising a polymer.

Agomelatine used in the composition according to the present invention is preferably in crystalline form. Preferably, agomelatine in crystalline form of polymorphic form X or form I is used.

A pharmaceutical composition can comprise agomelatine which is or comprises one of agomelatine polymorphic form X, agomelatine polymorphic form I, solvates of agomelatine, and mixtures thereof, optionally said agomelatine can be free of agomelatine polymorphic form II or can comprise agomelatine polymorphic form II in an amount of less than 10 wt.%, preferably of less than 5 wt.-%, more preferably of less than 3 wt.%, based on the total weight of agomelatine. In particular, the pharmaceutical composition can comprise agomelatine which is or comprises agomelatine polymorphic form X or agomelatine polymorphic form I.

The present inventors surprisingly found that the use of a pH modifier, stabilizes agomelatine metastable polymorphic form in a pharmaceutical composition. Furthermore, it has been surprisingly found that the presence of both a polymer and a pH modifier, contributes to stabilizing agomelatine in a pharmaceutical composition (e.g. contributes to agomelatine stabilization in view of the conversion of agomelatine X or I in an agomelatine form II having a different solubility in water, and/or in view of the formation of agomelatine degradation products).
Solvates can be in particular crystal complexes composed of at least two neutral molecules bound together in a crystal lattice, especially by non-covalent interactions. If one of the constituents is liquid at ambient temperature (20°C, especially at a pressure of 101325 Pa) the molecular complex can be in particular referred to as a solvate.

A pH modifier can be in particular a compound or a mixture of compounds. The pH modifier which, when 1 g pH modifier is mixed with 100 g of water at 20 °C provides a mixture, which has at 20°C a pH below below 5, and preferably below 3. Optionally, the pH modifier which, when 1 g pH modifier is mixed with 100 g of water at 20 °C can provide a mixture, which has at 20°C a pH of more than 1, optionally more than 2. If not explicitly disclosed to the contrary, the pH modifier is defined as an acidic substance, i.e. acidic pH modifier.

The mixing in water (water being purified water according to monograph in Ph. Eur.) can be in particular carried out at a temperature of 20°C, especially at a pressure of 101325 Pa. The mixing of the pH modifier with water can be carried out by adding 1 g of pH modifier to 100 g of water at a temperature of 20°C, especially at a pressure of 101 325 Pa, and agitating (especially stirring) until the pH modifier is completely dissolved or until the amount of dissolved pH modifier remains constant in the mixture comprising water and pH modifier. Undissolved pH modifier can be separated before pH measurement from the mixture after having obtained a constant concentration of dissolved pH modifier. pH can be in particular determined by potentiometric determination of pH, e.g. according to European Pharmacopoeia 9.2, chapter 2.2.3, "Potentiometric determination of pH".

The pH modifier can be selected from excipients with different functions as pharmaceutically acceptable excipients, e.g. can be selected from diluents, binders, disintegrants, lubricants, glidants, antitacking agents, and flavours, and mixtures thereof, which are in particular capable of modifying pH of the microenvironment of agomelatine particles (alone or in intimate junction, such as and intimate mixture, with at least one excipient) in the pharmaceutical composition and during its storage. Preferably, the pH modifier is selected from the group of pharmaceutically acceptable organic acids having at least one carboxylic functional group in their molecule, such as tartaric acid, acetic acid, oxalic acid. In particular, the pharmaceutical composition can comprise agomelatine which is in contact with pH modifier (and optionally further is in contact with polymer), e.g. is present in a mixture (especially initimate mixture) comprising both agomelatine and pH modifier (and optionally further comprising polymer). Intimate mixture means that particles of agomelatine are in at least partial direct contact with the particles of at least pH modifier or in another embodiment of the present invention are in at least partial direct contact with the particles of pH modifier and optionally with particles of polymer. In yet another embodiment agomelatine particles in intimate contact with particles of pH modifier are partially or completely covered by the polymer. In another embodiment of the present invention particles of agomelatine optionally admixed with polymer are partially or completely covered by pH modifier in solid state.

In particular, the pH modifier (which can be particularly a pharmaceutically acceptable pH modifier) can be selected from the group of acids and mixtures thereof, especially from the group of organic acids, inorganic acids, and mixtures thereof. The acid can be in particular selected from organic acids, inorganic acids, and mixtures thereof. An organic acid can be in particular selected from compounds comprising at least one (e.g. one or two or more) carboxylic acid moiety, compounds comprising at least one (e.g. one or two or more) sulfonic acid moiety, and mixtures thereof; preferably, an organic acid has a total number of carbon atoms of at least 2, preferably at least 4, and up to 20, preferably up to 16, more preferably up to 10.
Organic acids can be for example selected from compounds of formula (II), compounds of formula (III), compounds of formula (IV), compounds of formula (V), and mixtures thereof:

R¹-COOH (II)

R²-SO₃H (III)

HOOC-R³-COOH (IV)

HO₃S-R⁴-SO₃H (V)

wherein R¹ and R² can be independently selected from linear or branched C₁-C₂₀ alkyl (preferably linear or branched C₁-C₁₅ alkyl, especially linear or branched C₂-C₉ alkyl), optionally substituted with one or more substituents selected from -OH, -COOH, -SO₃H, and C₁-C₄-alkyl; C₆-C₁₂ cycloalkyl, optionally substituted with one or more substituents selected from -OH, -COOH, -SO₃H, and C₁-C₄-alkyl; linear or branched C₁-C₂₀ alkenyl, optionally substituted with one or more substituents selected from -OH, -COOH, -SO₃H, and C₁-C₄-alkyl; C₆-C₁₂ aryl, optionally substituted with one or more substituents selected from -OH, -COOH, -SO₃H, and C₁-C₄-alkyl;
wherein R³ and R⁴ can be independently selected from linear or branched C₁-C₂₀ alkylene (preferably linear or branched C₁-C₆alkylene), optionally substituted with one or more substituents selected from -OH, -COOH, -SO₃H, and C₁-C₄-alkyl; C₆-C₁₂cycloalkylene, optionally substituted with one or more substituents selected from -OH, -COOH, -SO₃H, and C₁-C₄-alkyl; and C₆-C₁₂ arylene, optionally substituted with one or more substituents selected from -OH, -COOH, -SO₃H, and C₁-C₄-alkyl. Preferably, R³ and R⁴ can be independently selected from linear or branched C₁-C₆alkylene (especially a C₁ or C₂ or C₃ or C₄ or C₅ alkylene), which is unsubstituted or which is substituted with at least one (e.g. 1, 2, 3, 4) -OH substituent, and is optionally further substituted with at least one -COOH substituent.

The polymer can be a molecule, the structure of which comprises a multiple repetition of units. A polymer may in particular refer to a molecule comprising a monomer chain comprising at least 6, preferably at least 10, especially at least 16 monomer units. The number average molecular weight of the polymer can be for example at least about 1,000 g/mol, further e.g. at least about 2,000 g/mol, in particular at least about 4,000 g/mol.

The polymer can be selected from excipients with different functions as pharmaceutically acceptable excipients. In particular the polymer can be selected from diluents, binders, disintegrants, lubricants, glidants, antitacking agents, bioadhesive materials, coating agents, controlled-release agents, dispersing agents, dissolution enhancers, emulsifying agents, emulsion stabilizers, extended-release agents, film-forming agents, foaming agents, granulation aids, modified-release agents, mucoadhesives, release-modifying agents, solubilizing agents, stabilizing agents, suspending agents, sustained-release agents, binders, thickening agents and viscosity-increasing agents, and mixtures thereof. Polymer can possess multiple function(s), i.e. it can act as pharmaceutically acceptable excipient with more than one function as described above.

Preferably, the polymer(s) act as stabilizing agent(s) and binder(s), which is(are)capable to coat the agomelatine particles, in particular on microenvironmental level, (alone or in intimate junction, such as an intimate junction, with at least one excipient). Preferably, polymer is or comprises polymer selected from the group of cellulose ethers with methoxyl substitution 28 to 30% and hydroxypropoxyl substitution 7 to 12%, e.g. hydroxypropylmethylcellulose. More preferably, viscosity grade of the specific polymer (2% in water at 20°C) is below 100 mPa·s, preferably in the range of 2-60 mPa·s, more preferably in the range 12-18 mPa·s.

In an embodiment, the polymer is or comprises polymer which is a water-soluble polymer, especially a water-soluble cellulose ether. Water-soluble polymer can be in particular polymer that dissolves in water or disperses in water to give a colloidal dispersion at a temperature of 20°C (especially at a pressure of 101 325 Pa), in particular wherein a polymer is dispersed in water in a quantity at least 1 mg of polymer per 1 ml of water at a temperature of 20°C (especially at a pressure of 101 325 Pa). In another embodiment, a water-soluble polymer can dissolve in water and has a solubility of more than 1 mg per 1 L of water at a temperature of 20°C (especially at a pressure of 101 325 Pa).

In an embodiment, the polymer can be selected from the group of cellulose ethers, and mixtures thereof; optionally, the cellulose ethers, and mixtures thereof can be water-soluble.
In particular, cellulose ether can be selected from alkyl celluloses, hydroxyalkyl celluloses, hydroxyalkyl alkyl celluloses. In particular, cellulose ethers can be selected from the group consisting of hydroxypropyl methylcellulose or hypromellose (HPMC), methylcellulose (MC), ethylcellulose (EC), hydroxyethyl cellulose (HEC), hydroxypropyl cellulose or hyprolose (HPC), hydroxypropyl ethylcellulose (HPEC), ethylhydroxyethylcellulose (EHEC), hydroxyethyl methylcellulose (HEMC), and mixtures thereof. Preferably, cellulose ether, e.g. hydroxypropyl methylcellulose or hypromellose, is in accordance with the respective definition provided in European Pharmacopoeia 9.2.

The skilled person may choose the degree of substitution and the molecular weight of the polymer(s), especially cellulose ether(s), on the basis of the general knowledge in the art. The molecular weight of the cellulose ether can be e.g. in the range of about 4000 to about 1 600 000 g/mol, in particular of about 10 000 to about 220 000 g/mol.

Especially, the cellulose ether can be selected from hydroxypropyl methylcellulose (HPMC), and mixtures of hydroxypropyl methylcellulose with one or more cellulose ethers other than HPMC. Mixtures of hydroxypropyl methylcellulose and one or more cellulose ethers other than HPMC can comprise at least 50 wt.-%, especially at least 85 wt.-% of HPMC, based on the total weight of the mixture of hydroxypropyl methylcellulose and cellulose ether(s) other than HPMC.

Hydroxypropyl methyl cellulose can have a percentage content of hydroxypropoxy groups in the range of 4 to 12, preferably in the range of 7 to 12, and a percentage content of methoxy groups in the range of 19 to 32, preferably in the range of 28 to 30. The term percentage content, e.g. of alkoxy groups and of hydroxyalkoxy groups, can have in particular the meaning of weight percentage content. The percentage content of alkoxy groups (e.g. methoxy groups) and hydroxyalkoxy groups (e.g. hydroxypropoxy groups) may be determined according to procedures known in the art, e.g. according to European Pharmacopoeia 9.2. For example, the percentage content of methoxy groups and hydroxypropoxy groups, respectively, can be determined according to European Pharmacopoeia 9.2, in particular section "Hypromellose".

The polymer can be a polymer that has a viscosity in the range from 2 to 140 000 mPa·s, preferably 2 to 60 mPa·s, most preferably from 12 to 18 mPa·s in a 2 weight percent aqueous solution at 20°C (especially at 20°C, and a pressure of 101325 Pa). In particular, a polymer, which is a cellulose ether or a mixture of cellulose ethers (especially which is HPMC or a mixture of HPMC and other cellulose ether(s), can have a viscosity ranging from about 2 to 60 mPa·s, preferably from 12 to 18 mPa·s in a 2 weight percent aqueous solution at 20°C. Viscosities may be measured by conventional methods, for example, by measuring the viscosity of an aqueous solution of the polymers at the desired concentration in a device for determining viscosity (e.g. Ubbelohde capillary tubes) at the specified temperature. In particular, viscosity may be determined according to European Pharmacopoeia 9.2, in particular according to chapter 2.2.9 "Capillary viscometer method".

A pharmaceutical composition of the present invention can comprise or can be granulate comprising agomelatine, preferably the granulate being obtainable by wet granulation; optionally said granulate comprising agomelatine can in particular further comprise a pH modifier and optionally a polymer and optionally further excipients, especially said granulate can comprise a pH modifier and a polymer and optional further excipients. Wet granulation procedures are known to the skilled person; in particular, wet granulation can be a granulation comprising contacting a compound or mixture to be granulated (during and/or before granulation) with a liquid.

In particular, the pharmaceutical composition can comprise: granulate comprising agomelatine, and at least one extragranular excipient; optionally said granulate comprising agomelatine can in particular further comprise a pH modifier and optionally a polymer and optionally further excipients, especially said granulate can comprise a pH modifier and a polymer and optional further excipients.

In an embodiment of the present invention, pH modifier, or polymer and pH modifier, can be incorporated completely intragranularly or completely extragranularly, or can be divided in between intragranular and extragranular phase, in particular in any ratio.

The term "intragranularly" can refer to an intragranular phase, such as granules, wherein agomelatine is granulated with an excipient or a mixture of excipients or wherein an excipient, or a mixture of excipients is granulated without agomelatine. The term "extragranularly" can relate to the additives added to the granulate, which additives can be excipient(s) with or without agomelatine, or just agomelatine.

In an embodiment, the pharmaceutical composition of the invention comprises granulate and an extragranular phase. The granulate or extragranular phase or both, granulate and extragranular phase, can comprise a polymer. In particular, in a pharmaceutical composition comprising granulate and an extragranular phase, the granulate or extragranular phase or both, granulate and extragranular phase, can comprise a polymer and a pH modifier.

In particular, the pharmaceutical composition can be
(A) a pharmaceutical composition, in particular an optionally coated comprimate (especially an optionally coated tablet), which can comprise
   granulate comprising agomelatine, and optionally pH modifier; and
   at least one extragranular excipient, and optionally (extragranular) agomelatine,
   preferably the pharmaceutical composition further comprises a polymer (said polymer can be an intragranular excipient, an extragranular excipient or can be both an intragranular excipient and an extragranular excipient (in any ratio)),
   or
(B) a pharmaceutical composition, in particular an optionally coated comprimate (especially an optionally coated tablet), which can comprise
   granulate comprising at least one excipient and optionally comprising agomelatine or being free of agomelatine; and
   at least one extragranular excipient, and (extragranular) agomelatine,
   preferably the pharmaceutical composition further comprises a pH modifier, and optionally a polymer (said polymer can be an intragranular excipient, an extragranular excipient or can be both an intragranular excipient and an extragranular excipient (in any ratio)).

Especially, the pharmaceutical composition can be
(A') a pharmaceutical composition, in particular an optionally coated comprimate (especially an optionally coated tablet), which can comprise
   granulate comprising agomelatine, and a pH modifier; and
   at least one extragranular excipient, and optionally (extragranular) agomelatine,
   wherein optionally the at least one extragranular excipient is free of a pH modifier or comprises a pH modifier, optionally the pharmaceutical composition further comprises a polymer (said polymer can be an intragranular excipient, an extragranular excipient or can be both an intragranular excipient and an extragranular excipient (in any ratio)).

In an embodiment, the pharmaceutical composition can be:
(B') a pharmaceutical composition, in particular an optionally coated comprimate (especially an optionally coated tablet), can comprise
   granulate comprising at least one excipient and optionally comprising agomelatine or being free of agomelatine; and
   at least one extragranular excipient, and (extragranular) agomelatine, the at least one extragranular excipient is or comprises a pH modifier
   wherein optionally the pharmaceutical composition further comprises a polymer (said polymer can be an intragranular excipient, an extragranular excipient or can be both an intragranular excipient and an extragranular excipient (in any ratio)).

In an embodiment, the pharmaceutical composition can be
(A") a pharmaceutical composition, in particular an optionally coated comprimate (especially an optionally coated tablet), which can comprise
   granulate comprising agomelatine, a pH modifier, and a polymer and optionally at least one further excipient; and
   at least one extragranular excipient, and optionally (extragranular) agomelatine,
   wherein optionally the at least one extragranular excipient is free of a pH modifier or comprises a pH modifier.

In an embodiment, the pharmaceutical composition can be
(B") a pharmaceutical composition, in particular an optionally coated comprimate (especially an optionally coated tablet), can comprise
   granulate comprising at least one excipient and optionally comprising agomelatine or being free of agomelatine; and
   at least one extragranular excipient, and (extragranular) agomelatine,
   wherein the at least one extragranular excipient comprises a pH modifier and a polymer.

In pharmaceutical compositions comprising granulate, e.g. in any one of pharmaceutical compositions (A), (B), (A'), (B'), (A"), (B"), the granulate can furthermore comprise one or more further excipients, in particular at least one of diluent, binder, disintegrant, lubricant, glidant, and antitacking agent, especially diluent and optionally at least one of binder, disintegrant, lubricant, glidant, and antitacking agent.

In another embodiment, the pharmaceutical composition of the present invention comprises agomelatine having average particle size between 1 and 200 µm. The average particle size is determined by laser method using a Malvern Mastersizer.

The term "average particle size" as used herein refers to volume mean diameter of particles. The diameter and volume mean diameter can be determined by laser light scattering using e.g. a Malvern Matersizer Aparatus. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer instrument. Usually, 100-800 mg of substance is dispersed in 5-10 mL of dispersant. Average particle size of agomelatine is determined by Malvern Mastersizer using Isopar L (viscosity at 25°C: 1.64 cSt (1.64 mm²/sec), saturated with agomelatine, as the dispersant medium.

A preferred pharmaceutical composition of the present invention is a solid pharmaceutical composition. The composition can be in a form selected from the group of tablets, granules, granulates, minitablets, microtablets, coated tablets, coated minitablets, coated microtablets, pills, powders, lozenges, sachets, soft and hard gelatine capsules, suppositories, etc., and mixtures thereof. Tablets, minitablets, microtablets, coated tablets, coated minitablets, coated microtablets, pills, granules, granulates, powders can be filled into sachet(s), capsules, e.g. soft or hard gelatine capsules. The pharmaceutical composition of the present invention is preferably suitable for oral application. Thus, it is preferred that the pharmaceutical composition is an oral dosage form such as a tablet or a coated tablet. The pharmaceutical composition can also be a tablet core, granules or granulate.

The pharmaceutical composition of the present invention is preferably formulated in a unit dosage form, each unit dosage form containing about 10 to about 100 mg of agomelatine, more preferably about 12.5 to about 50 mg of agomelatine. The term "agomelatine" can relate to agomelatine in crystalline form, preferably in crystalline form I or X, and most preferably in polymorphic form X. Agomelatine in polymorphic form X, or polymorphic form I may be prepared according to any process known in the art. In one embodiment agomelatine is agomelatine which is free of agomelatine form II. In particular agomelatine can be agomelatine obtainable by a process for the preparation of polymorphic form X of agomelatine, said process comprising: providing agomelatine, crystallizing agomelatine in the presence of at least one of an acid and a salt thereof, said crystallizing of agomelatine in the presence of at least one of an acid and a salt thereof comprising:
a) dissolving agomelatine in a first solvent to obtain a solution, said first solvent being or comprising an organic solvent,
b) preparing a mixture comprising (i) at least one of an acid and a salt thereof, and (ii) a second solvent, the at least one of an acid and a salt thereof being dissolved in the second solvent, and the second solvent being water or a solvent mixture comprising water,
c) adjusting the temperature of the mixture comprising (i) at least one of an acid and a salt thereof and (ii) a second solvent to a temperature in the range from 15°C to 40°C,
d) combining the solution comprising agomelatine with the mixture having a temperature in the range from 15°C to 40°C and comprising i) at least one of an acid and a salt thereof, and ii) a second solvent, e) isolating crystals of polymorph form X of agomelatine, and f) optionally drying the isolated crystals of polymorph form X of agomelatine.

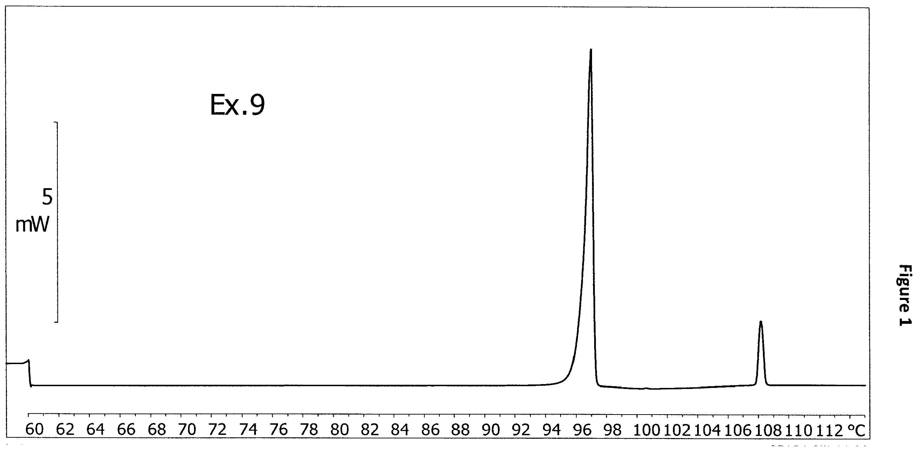

Agomelatine polymorph form X, especially agomelatine polymorph form X obtainable by the above-indicated process for the preparation of polymorphic form X of agomelatine comprising e.g. steps a) to f), can be for example characterized by a DSC thermogram showing an (endothermic) peak with peak T onset at 95-97 °C (preferably at 96.3 °C), optionally with a melting enthalpy of 100 to 130 J/g (preferably 116 J/g), which DSC thermogram can still further show a peak with peak T onset at 107 to 109°C (preferably at 107.9°C), optionally with a melting enthalpy (Δ H) of less than 60J/g; the DSC thermogram being measured when agomelatine polymorph form X is subjected to heating (e.g. starting from a temperature of 20°C) at a heating rate of 1°C /minute by a differential scanning calorimeter. Fig. 1 shows a DSC thermogram of agomelatine polymorph form X, which has been determined as indicated in the chapter "METHODS" below. Especially, agomelatine polymorph form X can be characterized by a DSC thermogram substantially as shown in Fig. 1 (especially in the range up to 103°C).

Agomelatine polymorphic form X can be agomelatine in the polymorphic form disclosed in EP 2 474 522 A1, especially in the claims (especially claims 1, 2) thereof. Table 1 summarizes data disclosed in EP 2 474 522 A1 for polymorphic form X:

**Table 1**

| **Analytic data for Agomelatine polymorphic form X (according to** EP 2 474 522 A1**)** | | |
|---|---|---|
| | | |
| 7 . X-ray powder diffraction: | 2θ | I/Iₒ |
| Instruments: Rigaku, Japan, D/MAX 2500 X-ray diffractometer | 12. 84 | 25 |
| Target: Cu-Ka radiation(λ= 1.5405), 2θ=2-40°C | 13. 84 | 23 |
| Step angle : 0.04°C | 16. 14 | 37 |
| Calculated time: 0.5 s | 18.56 | 100 |
| Tube voltage: 40KV | 19. 12 | 27 |
| Tube current: 100mA | 20. 86 | 75 |
| Scanning speed: 8°C/min | | |
| Filter: Graphite monochromator | 21.20 | 30 |
| Error of 2θ value: 2θ value±0.10 | 23.84 | 64 |
| | | |
| Infrared spectroscopy (IR): | | |
| Instruments: Nicolet, US, MAGNA-560 Fourier transform infrared spectrometer. The wave numbers of IR spectrum for the agomelatine crystal (...) using potassium bromide for grinding are as follows: characteristic absorption peaks at 3234, 3060, 2940, 1638, 1511, 1436, 1249, 1215, 1184, 1032, 908, 828, 755, 588 cm⁻¹ . | | |

In particular, a crystal of agomelatine polymorphic form X can be characterized by one or more of: X-ray powder diffraction pattern in the 2Θ using Cu-Kα radiation having characteristic diffraction peaks at 12.84, 13.84, 16.14, 18.56, 19.12, 20.86, 21.20, 23.84, and IR absorption pattern having characteristic absorption peaks at 3234, 3060, 2940, 1638, 1511, 1436, 1249, 1215, 1184, 1032, 908, 828, 755, 588 cm⁻¹.

Agomelatine polymorphic form I can be agomelatine in the polymorphic form I disclosed in WO 2013/018100 A1 or WO 2011/054917 A1. In particular, a crystal of agomelatine polymorphic form I can be characterized by one or more of: XRPD with characteristic peaks at about 10.8, 11.2, 11.9, 13.8, 14.6, 14.9, 15.4, 15.8, 17.0, 17.5, 18.4, 19.5, 19.8, 20.5, 21.0, 21.3, 21.8, 22.6, 23.0, 24.0, 24.6, 25.4, 26.0, 26.4, 27.1, 27.7, 28.8, 30.1, 31.6, 31.9, 33.0, 33.7, 34.5 and 36.0 ± 0.2 degrees two-theta, (wherein the X-ray source can be copper Kα) (XRPD data according to WO 2013/018100 A1), and differential scanning calorimetry (DSC) with an endotherm curve at about 108.62°C measured at 0.2 °C/min. ramp, in particular substantially in accordance with Fig. 2 of WO 2013/018100 A1. Polymorph form I of agomelatine can be characterized by the crystal data disclosed in WO 2011/054917 A1 and in Acta Cryst. (1994), C50, 907-910 (authors: B. Tinant, J.-P. Declercq). In particular, polymorph form I of agomelatine can be characterized by the following crystal data (wherein Z can be the number of molecules in one cell):
C₁₅H₁₇NO₂
Mr = 243.30
Orthorhombic
Pca2₁
a=31.501 (4) A
b = 9.5280 (10)A
c = 17.906 (2) Å
Z= 16

Agomelatine polymorphic form II can be agomelatine in the polymorphic form disclosed in EP 1 564 202 B1, especially claim 16 thereof. In particular, a crystal of agomelatine polymorphic form II can be characterized by one or more of the following data, obtained from a powder diagram, in particular obtained from
the powder diagram produced using a Bruker AXS D8 high-resolution diffractometer having an angular field 2θ of 3°-90°, a step of 0.01 ° and 30 s per step :
- monoclinic crystal lattice,
- lattice parameters : a = 20.0903 A, b = 9.3194 Å, c = 15.4796 Å, β = 108.667°
- space group : P2₁/n
- number of molecules in the lattice : 8
- lattice volume : V_{lattice} = 2746.742 A³
- density : d = 1.13 g/cm³.

The term "unit dosage form" refers to physically discrete unit(s) suitable as unitary dosages for human subjects and other mammals, each unit containing a predetermined quantity of agomelatine calculated to produce the desired therapeutic effect, in association with at least one suitable excipient. An immediate release composition of unit dosage form is preferred.

The pharmaceutically acceptable excipients used for the preparation of the pharmaceutical compositions of the present invention particularly include excipients with different functions. Excipients used for the preparation of pharmaceutical compositions of the present invention can be of pharmaceutical grade, i.e. their quality is appropriate for mammal use and intended for the preparation of solid pharmaceutical compositions. The pharmaceutical composition of the present invention can comprise at least one excipient selected from the group of diluents, binders, disintegrants, lubricants, glidants and antitacking agents. In particular, a pharmaceutical composition can comprise more than one excipient selected from the groups of diluents, binders, disintegrants, lubricants, glidants and antitacking agents. Other and further excipients with different functions, e.g. polymers, pH modifiers, antioxidants, surfactants etc. can be included in the pharmaceutical compositions of the present invention, or one or more excipients with different functions can be excluded from the composition. Furthermore, excipients can have multiple functions, i.e. one excipient can be used as a binder/diluent, etc.

The pharmaceutical composition of the present invention may comprise diluent. In a pharmaceutical composition comprising granulate and optionally at least one extragranular excipient, the granulate may comprise diluent, or the at least one extragranular excipient may comprise diluent, or both granulate and the at least one extragranular excipient may comprise diluent. In the present application the term diluent may refer to one or more diluents, and thus encompasses one diluent, as well as a mixture of diluents. Particularly suitable diluent can be selected from carbohydrates or its derivatives, such as lactose, e.g. lactose monohydrate, anhydrous lactose, spray dried and/or granulated lactose, sucrose, fructose, dextrates, saccharose, raffinose, trehalose, fructose and mixtures thereof, dextrin, sugar alcohols such as xylitol, mannitol, maltitol, isomalt, sorbitol, celluloses, in particular powdered cellulose, microcrystalline cellulose, silicified microcrystalline cellulose, starch and derivatives thereof, in particular low moisture starch, starch such as maize starch, potato starch, rice starch, tapioca starch or wheat starch, pregelatinised starch, low moisture pregelatinised starch, magnesium aluminometasilicate, such as Neusilin, calcium salts of phosphoric acid, such as calcium hydrogen phosphate (in anhydrous or hydrate form), alkali carbonates, earth alkali carbonates, alkali hydrogencarbonates, earth alkali hydrogencarbonates, such as calcium carbonate, sodium carbonate or potassium carbonate or calcium hydrogencarbonate, sodium hydrogencarbonate or potassium hydrogencarbonate, and calcium lactate, and mixtures thereof. Preferably, diluent is preferably selected from microcrystalline cellulose, saccharides, such as lactose, polyols and corn starch, and mixtures thereof. More preferably, the pharmaceutical composition of the present invention comprises at least one diluent, in particular selected from lactose and starch (such as maize starch), and mixtures thereof. Especially, in a pharmaceutical composition which is or comprises granulate, the granulate may comprise diluent, in particular selected from the above-indicated group or diluent which is or comprises lactose or starch (such as maize starch) or a mixture of lactose and starch.

The pharmaceutical composition of the present invention can furthermore comprise binder. In a pharmaceutical composition comprising granulate and optionally at least one extragranular excipient, the granulate may comprise binder, or the at least one extragranular excipient may comprise binder, or both the granulate and the at least one extragranular excipient may comprise binder. In the present application the term binder may refer to one or more binders, and thus encompasses one binder, as well as a mixture of binders. The binder can be selected from the group of povidone (polyvinylpyrrolidone), copovidone (vinylpyrrolidone-vinyl copolymer), cellulose, in particular powdered cellulose, crystalline cellulose, microcrystalline cellulose, silicified microcrystalline cellulose, cellulose derivatives, such as hydroxymethylcellulose, hydroxyethylcellulose, hydroxypropylcellulose, low substituted hydroxypropylcellulose and hydroxypropylmethylcellulose or other cellulose ethers, starch, such as maize starch, potato starch, rice starch, tapioca starch or wheat starch, pregelatinised starch, α-starch or dextrin, gum arabic, pullulan, polymethacrylates, and mixtures thereof. Preferably, the dry binder can be selected from povidone, hydroxypropylcellulose, hydroxypropylmethylcellulose, starch such as maize starch, potato starch, rice starch, tapioca starch or wheat starch, and mixtures thereof. More preferably, the pharmaceutical composition of the present invention comprises at least one binder, selected from hydroxypropylmethylcellulose, and starch such as maize starch.

It is also preferred that the pharmaceutical composition of the present invention furthermore comprises disintegrant. In a pharmaceutical composition comprising granulate and optionally at least one extragranular excipient, the granulate may comprise disintegrant, or the at least one extragranular excipient may comprise disintegrant, or the granulate and the at least one extragranular excipient may comprise disintegrant. In the present application the term disintegrant may refer to one or more disintegrants, and thus encompasses one disintegrant, as well as a mixture of disintegrants. The disintegrant can be selected from crospovidone, starch such as maize starch, potato starch, rice starch, tapioca starch or wheat starch, pregelatinised starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, hydroxypropylcellulose, in particular low substituted hydroxypropylcellulose, sodium and/or calcium alginate, docusate sodium, methylcellulose, agar, guar gum, chitosan, alginic acid, and mixtures thereof. Preferably, disintegrant is selected from starch such as maize starch, potato starch, rice starch, tapioca starch or wheat starch, pregelatinised starch, sodium starch glycolate, and mixtures thereof. More preferably, the pharmaceutical composition of the present invention comprises at least one disintegrant, selected from sodium starch glycolate and starch such as maize starch.

The pharmaceutical composition of the present invention can furthermore comprise lubricant. In a pharmaceutical composition comprising granulate and optionally at least one extragranular excipient, the granulate may comprise lubricant, and/or the at least one extragranular excipient may comprise lubricant. In the present application the term lubricant may refer to one or more lubricants, and thus encompasses one lubricant, as well as a mixture of lubricants. The lubricant can be selected from the group of metal salts of fatty acids with 12 to 20 carbon atoms such as magnesium stearate, calcium stearate, aluminium stearate or zinc stearate, magnesium palmitate and magnesium oleate, fatty acids with 12 to 20 carbon atoms such as stearic acid, palmitic acid and oleic acid, hydrogenated vegetable oil, hydrogenated castor oil, talc, meads wax or spermaceti, boric acid, sodium stearylfumarate, macrogols, and mixtures thereof. Preferably, lubricant is selected from magnesium and/or calcium stearate, stearic acid, talc, and mixtures thereof. More preferably, the pharmaceutical composition of the present invention comprises at least one lubricant, selected from stearic acid and magnesium stearate.

The pharmaceutical composition of the present invention can furthermore comprise one or more glidants, selected from colloidal silica, talc and magnesium trisilicate, preferably colloidal silica. In a pharmaceutical composition comprising granulate and optionally at least one extragranular excipient, the granulate may comprise glidant, and/or the at least one extragranular excipient may comprise glidant.
The pharmaceutical composition of the present invention can furthermore comprise one or more antitacking agents, selected from talc, glyceryl monostearate and stearic acid, preferably stearic acid. In a pharmaceutical composition comprising granulate and optionally at least one extragranular excipient, the granulate may comprise antitacking agent; optionally also the at least one extragranular excipient may comprise antitacking agent.

The pharmaceutical composition of the present invention can be in solid dosage form such as a tablet or coated tablet and preferably comprises agomelatine and pH modifier, and optionally at least one further excipient selected from excipients with the function as defined: diluent, binder, disintegrant, lubricant, glidant and antitacking agent. In another embodiment of the present invention, the compositions in the solid dosage forms of the present invention preferably comprise agomelatine, at least one pH modifier and at least one polymer, and optionally at least one further excipient selected from excipients with the function as defined: diluent, binder, disintegrant, lubricant and glidant.

In an embodiment, the pharmaceutical composition of the present invention (which is preferably a solid dosage form, such as a tablet or coated tablet) can comprise agomelatine and a pH modifier and optionally a polymer, and optionally at least one further excipient selected from diluents, binders, disintegrants, lubricants, glidants and antitacking agents.

In an embodiment, the pharmaceutical composition of the present invention (which is preferably a solid dosage form, such as a tablet or coated tablet) can comprise agomelatine, a pH modifier, and a polymer, and optionally at least one further excipient selected from diluents, binders, disintegrants, lubricants, glidants and antitacking agents.

The polymer can be selected from excipients which are available for pharmaceutical use at one or different molecular weight as defined by manufacturers or in a representative literature such as pharmacopoeias. According to these data, they are defined with at least one of the following terms, known in the art: bioadhesive materials, coating agents, controlled-release agents, dispersing agents, dissolution enhancers, emulsifying agents, emulsion stabilizers, extended-release agents, film-forming agents, foaming agents, granulation aids, modified-release agents, mucoadhesives, release-modifying agents, solubilizing agents, stabilizing agents, suspending agents, sustained-release agents, binders, thickening agents and viscosity-increasing agents. The polymer can be selected from cellulose ethers.

Typical cellulose ethers are hydroxyethylcellulose, hydroxypropylcellulose,
hydroxypropylmethylcellulose, methylcellulose. Preferably, the polymer is selected from the group of cellulose ethers with methoxyl substitution 28 to 30% and hydroxypropoxyl substitution 7 to 12%, i.e. hydroxypropylmethylcellulose. More preferably, viscosity grade of the specific polymer (2% in water at 20°C) is 12-18 mPa·s.

During development, other stabilizers were used in order to stabilize physical polymorphic form of metastable forms, such as form X and/or form I of agomelatine, for example:
- hydroxypropylmethylcellulose with methoxyl substitution 19 to 24% and hydroxypropoxyl substitution 7 to 12% and viscosity grade 80-120 mPa·s (2wt% in water at 20°C);
- hydroxypropylmethylcellulose with methoxyl substitution 19 to 24% and hydroxypropoxyl substitution 7 to 12% and viscosity grade 2,663-4,970 mPa·s (2wt% in water at 20°C);
- hydroxypropylmethylcellulose with methoxyl substitution 28 to 30% and hydroxypropoxyl substitution 7 to 12% and viscosity grade 40-60 mPa·s (2wt% in water at 20°C);
- poly(butyl methacrylate, (2-dimethylaminoethyl) methacrylate, methyl methacrylate) 1 : 2 : 1, e.g. Eudragit E PO,
- poly(methacrylic acid, ethyl acrylate) 1 : 1, e.g. Eudragit L30D,
- carbomers, e.g. Carbopol 971P,
- glyceryl monostearate.

The pH modifier can be or can comprise pH modifier selected from excipients which are available for pharmaceutical use with different functions as pharmaceutically acceptable excipients. In particular, the pH modifier can be or can comprise pH modifier selected from diluents, binders, disintegrants, lubricants, glidants, antitacking agents, and flavours, and mixtures thereof. Preferably, pH modifier is selected from the group of pharmaceutically acceptable acids, such as 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid, in particular ascorbic acid (L), aspartic acid, in particular aspartic acid (L), benzenesulfonic acid, benzoic acid, camphoric acid, in particular camphoric acid (+),camphor-10-sulfonic acid, in particular camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid, in particular glucoheptonic acid (D), gluconic acid, in particular gluconic acid (D), glucuronic acid, in particular glucuronic acid (D), glutamic acid, glutaric acid, glycerophosphoric acid, glycolic acid, hippuric acid, hydrobromic acid, hydrochloric acid, isobutyric acid, lactic acid, in particular lactic acid (DL), lactobionic acid, lauric acid, maleic acid, malic acid, in particular malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid, in particular pyroglutamic acid (- L), salicylic acid, sebacic acid, succinic acid, sulfuric acid, tartaric acid, in particular tartaric acid (+ L), thiocyanic acid, toluenesulfonic acid, in particular toluenesulfonic acid (p) and undecylenic acid, and mixtures thereof.

In one embodiment, the pH modifier can be free of stearic acid. Furthermore, the pharmaceutical composition (e.g. granulate, comprimate, tablet core) can be free of stearic acid. Moreover, in a pharmaceutical composition comprising a granulate, the granulate can be free of stearic acid. In one embodiment, the pH modifier can be free of acids forming a co-crystal with agomelatine. Furthermore, the pharmaceutical composition can be free of agomelatine/acid co-crystal. Moreover, in a pharmaceutical composition comprising a granulate, the granulate can be free of agomelatine/acid co-crystal. Preferably, the pH modifier (which can be in particular an acid or a mixture of acids) can have a solubility in water at 20°C of more than 1 milligram of pH modifier per liter of water, preferably of more than 10 milligram of pH modifier per liter of water, optionally of more than 0,5 gram of pH modifier per liter of water, further optionally of more than 1 g, still further optionally of more than 5 g, of pH modifier per liter of water. In an embodiment, the solubility can be less than 10 000 milliliters of solvent per gram of the pH modifier (in particular of the acid or mixture of acids), as defined in descriptive level of solublity (see table below), e.g. the pH modifier can be from »very soluble« to »very slightly Soluble« as described General notices in European Pharmacopoeia 9.2..

**Table 2: Descriptive level of solubility referred to a temperature between 15°C and 25°C.**

| Descriptive term | Approximate volume of solvent in milliliters per gram of solute |
|---|---|
| Very soluble | less than 1 |
| Freely soluble | from 1 to 10 |
| Soluble | from 10 to 30 |
| Sparingly soluble | from 30 to 100 |
| Slightly soluble | from 100 to 1000 |
| Very slightly soluble | from 1000 to 10 000 |
| Practically insoluble | more than 10 000 |

Determining solubility forms part of the general knowledge in the art. In particular, solubility can be determined by the following method: The material to be tested (e.g. compound or mixture) can be added in surplus to the solvent (e.g. water) and agitated (e.g. shaken or stirred) for 72 hours. Presence of saturation can be confirmed by observation of the presence of un-dissolved material. After filtration of the slurry for removing un-dissolved material, a sample for analysis is taken. The amount of dissolved material can be determined by the skilled person by known methods, e.g. gravimetric methods, or ultraviolet/visible spectroscopy. All steps can be carried out at 20°C (especially at 20°C, and a pressure of 101 325 Pa).

In particular, the pH modifier (which can be in particular an acid or a mixture of acids) can be a pH modifier which, when 1 g pH modifier is mixed with 100 g of water at 20 °C provides a pH below 5, and preferably below 3, and has a solubility in water at 20°C of more than 1 milligram of pH modifier per liter of water, preferably of more than 10 milligram of pH modifier per liter of water, optionally of more than 0.5 gram of pH modifier per liter of water, further optionally of more than 1 g, still further optionally of more than 5 g, of pH modifier per liter of water.

The pH modifier can be one or more acids selected from acids having a pKa of about 5 or below, especially of about 4.9 or below, further especially of 4.8 or below (preferably having a pKa in the range from 0.5 to 5, further especially in the range from 1 to 4.9, especially from 2 to 4.8), and optionally further having a solubility in water at 20°C (in particular at 20°C and a pressure of 101 325 Pa) of more than 1 milligram (optionally of more than 10 milligram, optionally of more than 0.5 gram) per liter of water.

According to one embodiment, the pH modifier can be one or more acids selected from acids having a pKa below the pKa of stearic acid (especially selected from acids having the pKa of nonanoic acid or a lower pKa), and optionally further having a solubility in water at 20°C (in particular at 20°C and a pressure of 101325 Pa) of more than 1 milligram (preferably of more than 10 milligram, optionally of more than 0.5 gram) per liter of water. Optionally, the pH modifier can be one or more acids selected from acids having a pKa which is higher than the pKa of HCl, but is below the pKa of stearic acid (especially selected from acids having a pKa which is at least the pKa of benzenesulfonic acid and not more than the pKa of nonanoic acid), and optionally further having solubility in water at 20°C (in particular at 20°C and a pressure of 101 325 Pa) of more than 1 milligram (preferably of more than10 milligram, optionally of more than 0.5 gram) per liter of water.

The acid dissociation constant Ka is a constant defining the strength of an acid in solution. For practical purposes, it is more convenient to refer to the constant pKa, pKa being the negative decadic logarithm of Ka (pKa and pH are known in the art, and are explained e.g. in: P.W. Atkins, Physical Chemistry, 3rd . ed., Oxford University Press, chapter 12.4, (c) "pk and ph"; E. Riedel, Anorganische Chemie, de Gruyter, 1988, chapter 3.7). Dissociation constants of acids in aqueous solution are provided in Tables, especially in CRC Handbook of Chemistry and Physics, 51st. ed., 1970-1971, published by The Chemical Rubber Co., ed. R. C. Weast. Temperature for determining acid dissociation constant Ka can be in particular 25°C (especially at a pressure of 101 325 Pa). For determining pKa, the acid is dissolved in water. A possible method of determining the pKa of an acid can be carried out according to EP 1 169 015 B1, especially paragraphs [0032] to [0037] thereof. In case of polyprotic acids (i.e. acids that can lose more than one proton, such as e.g. tartaric acid), the term "pKa" can refer herein to the constant for dissociation of the first proton from the acid. This is the dissociation constant having the lowest value among the several dissociation constants determined for this polyprotic acid (for example, oxalic acid has pKa = 1.23, and (for the second (proton) dissociation step) pKa2 = 4.19, pKa values as indicated in: CRC Handbook of Chemistry and Physics, supra).

In a preferred embodiment of the present invention, the pharmaceutical composition comprises agomelatine and the following excipients:
tartaric acid, and hydroxypropylmethylcellulose,
preferably tartaric acid, hydroxypropylmethylcellulose, at least one or both of lactose, and starch (especially maize starch), more preferably lactose, starch (especially maize starch), sodium starch glycolate, hydroxypropylmethylcellulose, tartaric acid, stearic acid, magnesium stearate and colloidal silica.

In particular, a pharmaceutical composition of the present invention can be or can comprise granulate comprising agomelatine (preferably the granulate being obtainable by wet granulation), which granulate further comprises tartaric acid, and hydroxypropylmethylcellulose, preferably tartaric acid, hydroxypropylmethylcellulose, at least one or both of lactose, and starch (especially maize starch), more preferably lactose, starch (especially maize starch), hydroxypropylmethylcellulose, tartaric acid. Optionally the pharmaceutical composition can further comprise at least one extragranular excipient, selected from stearic acid, sodium starch glycolate, colloidal silica and magnesium stearate.

Solid dosage forms described above can be optionally coated with aqueous soluble film coating, having average thickness of at least 1 µm, measured by scanning electron microscopy (SEM) of crossection of coated solid dosage form. Optional film coating can comprise aqueous soluble polymer selected from cellulose ethers, such as hydroxypropylmethylcellulose (hypromellose) having viscosity of 2wt. -% aqueous solution less than 60 mPa·s, preferably less than 40 mPa·s and most preferably less than 20 mPa·s measured at 20°C. Other polymers that can be used for coating are selected from polyvinyl alcohol, povidone, sodium carboxymethyl cellulose, waxy materials, acrylic polymers, block polymer of polyvinyl alcohol and polyethylene glycol commercially available under trade name Kollicoat® IR and Kollicoat® Protect. Optionally, other excipients can be used and are selected from lubricants, antitacking agents, pigments, colourant and/or plasticizers.

Typical cellulose ethers used in film coatings (which can be used for coating the pharmaceutical compositions of the present invention) are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

Commonly used plasticizers (which can be used for coating the pharmaceutical compositions of the present invention) can be categorized into three groups: polyols (glycerol, propylene glycol, macrogols), organic esters (phthalate esters, dibutyl sebacate, citrate esters, triacetin), oils/glycerides (castor oil, acetylated monoglycerides, fractionated coconut oil).

Commonly used lubricants and/or antitacking agents (which can be used for coating the pharmaceutical compositions of the present invention) can be selected from the group of metal salts of fatty acids with 12 to 20 carbon atoms such as magnesium stearate, calcium stearate, aluminium stearate or zinc stearate, magnesium palmitate and magnesium oleate, fatty acids with 12 to 20 carbon atoms such as stearic acid, palmitic acid and oleic acid, hydrogenated vegetable oil, hydrogenated castor oil, talc, meads wax or spermaceti, boric acid, sodium stearyl fumarate, and mixtures thereof.

Colourants/opacifiers (which can be used for coating the pharmaceutical compositions of the present invention) are classified into several groups: organic dyes and their lakes, inorganic colours, natural colours. Pigments can be selected from metal oxides such as iron or titanium oxides.
Combination of different materials from each group can be combined, in particular in defined ratios.

Film coating suspensions can be used as ready-to-make preparations which are available on the market. Film coating dispersion can be prepared by using different solvents (water, alcohols, ketones, esters, chlorinated hydrocarbons), preferably water.

A composition of coating suspension (calculated on dry material) is particularly preferred which comprises:
- 1-99% by weight of polymer, preferably 1-95% of polymer,
- 1-50% by weight of plasticizer, preferably 1-40% of plasticizer,
- 0.1-20% by weight of lubricant, preferably 1-10% of lubricant,
- 0.1-20% by weight of colourant/opacifier and/or pigment, preferably 0.1-10% of colourant/opacifier and or/pigment.

The composition of the coating layer of the pharmaceutical composition of the present invention preferably comprises at least one excipient selected from excipients with the function as defined of polymer and plasticizer. In another embodiment of the present invention, the composition of the coating layer of the present invention preferably comprises at least one excipient selected from excipients with the function as defined polymer, plasticizer, lubricant and colourant/opacifier.

If not indicated otherwise, all percentages ("%") given herein are wt. %. Excipients mentioned herein, in particular pH modifier, polymer, can be pharmaceutically acceptable excipients. The term "pharmaceutically acceptable" as used herein can in particular indicate that the "pharmaceutically acceptable" compound or composition is suitable for administration to a subject to achieve a treatment and/or prevention of a disease, of a disorder or of a condition, without unduly deleterious side effects in light of the necessity of the treatment. The term "therapeutically effective amount" as used herein can in particular indicate an amount sufficient to ameliorate, reverse, stabilize, slow and/or delay progression of a disease, a disorder or a condition.

Preferably, the pharmaceutical composition of the present invention comprises agomelatine in an amount of 5-50wt.%, more preferably 10-40wt.%, most preferably 10-30wt.%, based on the total weight of the pharmaceutical composition. In a pharmaceutical composition of the present invention (e.g. comprimate, in particular tablet) comprising granulate and at least extragranular excipient, the granulate can comprise agomelatine in an amount of 5-50wt.%, more preferably 10-40wt.%, most preferably 10-30wt.%, each based on the total weight of the pharmaceutical composition.

In an embodiment, the pharmaceutical composition, in particular pharmaceutical composition for oral administration, comprises
agomelatine, in particular agomelatine in crystalline form, in an amount of 5-50 wt.%, more preferably 10-40 wt.%, most preferably 10-30 wt.%, and
a pH modifier in an amount of 1-40 wt.%, more preferably 1-30 wt.%, most preferably 1-25 wt.%, especially 3-25wt.%, further especially 5-16wt.%, and
optionally a polymer in an amount of 1-50 wt.%, more preferably 1-40 wt.%, most preferably 1-30 wt.%, especially 3-25wt.%, each based on the total weight of the pharmaceutical composition.

In a preferred embodiment, the pharmaceutical composition can be a tablet comprising or consisting of a tablet core, especially a coated tablet which can comprise a tablet core and a coating coated on the tablet core (which coating can partially or completely cover the tablet core). In an embodiment, the pharmaceutical composition, in particular pharmaceutical composition for oral administration, can be a tablet comprising or consisting of a tablet core, wherein the tablet core comprises agomelatine, in particular agomelatine in crystalline form, in an amount of 5-50 wt.%, more preferably 10-40 wt.%, most preferably 10-30 wt.%, and
a pH modifier in an amount of 1-40 wt.%, more preferably 1-30 wt.%, most preferably 1-25 wt.%, especially 3-25wt.%, further especially 5-16wt.%, and
optionally a polymer in an amount of 1-50 wt.%, more preferably 1-40 wt.%, most preferably 1-30 wt.%, especially 3-25wt.%, each based on the total weight of the tablet core.

In a pharmaceutical composition of the present invention (e.g. granulate, comprimate, in particular tablet) which is granulate or comprises granulate and optionally at least one extragranular excipient, the granulate can comprise agomelatine in an amount of 5-50wt.%, more preferably 10-40wt.%, most preferably 10-30wt.%, based on the total weight of the pharmaceutical composition. Especially, in a pharmaceutical composition of the present invention which is granulate or which comprises granulate and optionally at least extragranular excipient, the granulate can comprise agomelatine, in particular agomelatine in crystalline form, in an amount of 5-50 wt.-%, more preferably 10-40 wt.%, most preferably 10-30 wt.%, and a pH modifier in an amount of 1-40 wt.%, more preferably 1-30 wt.%, most preferably 1-25 wt.%, especially 3-25wt.%, further especially 5-16wt.%, and optionally a polymer in an amount of 1-50 wt.%, more preferably 1-40 wt.%, most preferably 1-30 wt.%, especially 3-25wt.%, and optionally one or more further excipients, each based on the total weight of the pharmaceutical composition.

Particularly advantageous can be a pharmaceutical composition comprising agomelatine (in particular in an amount of 5-50 wt.-%, more preferably 10-40 wt.%, most preferably 10-30 wt.%, based on the total weight of the pharmaceutical composition), and pH-modifier (in particular in an amount 1-40 wt.%, more preferably 1-30 wt.%, most preferably 1-25 wt.%, especially 3-25 wt.%, further especially 5-16 wt.%, based on the total weight of the pharmaceutical composition), in which composition pH modifier and agomelatine may be present in a weight ratio between pH modifier and agomelatine (pH modifier: agomelatine), which can be from 1:10 to 10:1, preferably 1:5 to 5:1, more preferably 1:2 to 2:1, especially 1:1.6 to 1.6:1, most preferably about 1:1.6.

Furthermore, particularly advantageous can be a pharmaceutical composition comprising agomelatine (in particular in an amount of 5-50 wt.-%, more preferably 10-40 wt.%, most preferably 10-30 wt.%, based on the total weight of the pharmaceutical composition), pH modifier in particular in an amount 1-40 wt.%, more preferably 1-30 wt.%, most preferably 1-25 wt.%, especially 3-25 wt.%, further especially 5-16 wt.%, based on the total weight of the pharmaceutical composition) and polymer (in particular in an amount of 1-50 wt.%, more preferably 1-40 wt.%, most preferably 1-30 wt.%, especially 3-25 wt.%, based on the total weight of the pharmaceutical composition), wherein the weight ratio between polymer and agomelatine (polymer: agomelatine) can be from 1:10 to 10:1, preferably 1:5 to 5:1, more preferably 1:5 to 2:1, most preferably 1:3 to 1.5:1.

Furthermore, particularly advantageous can be a pharmaceutical composition comprising agomelatine (in particular in an amount of 5-50 wt.-%, more preferably 10-40 wt.%, most preferably 10-30 wt.%, based on the total weight of the pharmaceutical composition), pH modifier in particular in an amount 1-40 wt.%, more preferably 1-30 wt.%, most preferably 1-25 wt.%, especially 3-25 wt.%, further especially 5-16 wt.%, based on the total weight of the pharmaceutical composition) and polymer (in particular in an amount of 1-50 wt.%, more preferably 1-40 wt.%, most preferably 1-30 wt.%, especially 3-25 wt.%, based on the total weight of the pharmaceutical composition), wherein the weight ratio between polymer and pH modifier (polymer: pH modifier) can be from 1:10 to 10:1, preferably 1:5 to 5:1, more preferably 1:4 to 4:1, most preferably 1:3 to 3:1.

Furthermore, particularly advantageous can be a pharmaceutical composition comprising agomelatine (in particular in an amount of 5-50 wt.-%, more preferably 10-40 wt.%, most preferably 10-30 wt.%, based on the total weight of the pharmaceutical composition), pH modifier in particular in an amount 1-40 wt.%, more preferably 1-30 wt.%, most preferably 1-25 wt.%, especially 3-25 wt.%, further especially 5-16 wt.%, based on the total weight of the pharmaceutical composition) and polymer (in particular in an amount of 1-50 wt.%, more preferably 1-40 wt.%, most preferably 1-30 wt.%, especially 3-25 wt.%, based on the total weight of the pharmaceutical composition), wherein the weight ratio between polymer, pH modifier and agomelatine (polymer: pH modifier: agomelatine) can be 0.1-5 : 0.1-5 : 1, preferably 0.1-3 : 0.1-3 : 1, more preferably 0.1-2 : 0.1-2 : 1, most preferably 0.3-1.5 : 0.5-1 : 1.

According to a more preferred embodiment, the pharmaceutical composition of the present invention (which can be e.g. a granulate, a coated or uncoated comprimate (such as e.g. tablet, tablet core, etc.) comprises:
agomelatine (in particular agomelatine in crystalline form): 5-50wt.%, more preferably 10-40wt.%, most preferably 10-30wt.%;
diluent: 10-90wt.%, more preferably 20-80wt.%, most preferably 20-60wt.%;
binder: 1-50wt.%, more preferably 1-40wt.%, most preferably 1-30wt.%;
disintegrant: 1-40wt.%, more preferably 1-30wt.%, most preferably 1-25wt.%;
lubricant: 1-20wt.%, more preferably 1-15wt.%, most preferably 1-10wt.%;
glidant: 0.1-20wt.%, more preferably 0.1-15wt.%, most preferably 0.1-10wt.%; antitacking agent: 1-20wt.%, more preferably 1-15wt.%, most preferably 1-10wt.%;
polymer: 1-50wt.%, more preferably 1-40wt.%, most preferably 1-30wt.%, especially 3-25wt.%;
pH modifier: 1-40wt.%, more preferably 1-30wt.%, most preferably 1-25wt.%, especially 3-25wt.%, further especially 5-16wt.%, each based on the total weight of the pharmaceutical composition (and optionally the composition can comprise one or more further excipients). The term "polymer" refers to "optional polymeric material" of the present invention.

The total sum of the components of a composition of the present invention can be 100%.

According to one embodiment, the pharmaceutical composition of the present invention is a coated comprimate comprising a comprimate and a coating coated on the comprimate, (e.g. a coated tablet comprising a tablet core and a coating coated on the tablet core), wherein the comprimate comprises:
agomelatine (in particular agomelatine in crystalline form): 5-50wt.%, more preferably 10-40wt.%, most preferably 10-30wt.%;
polymer: 1-50wt.%, more preferably 1-40wt.%, most preferably 1-30wt.%, especially 3-20wt.%;
pH modifier: 1-40wt.%, more preferably 1-30wt.%, most preferably 1-25wt.%, especially 3-25wt.%, further especially 5-16wt.%, each based on the total weight of the pharmaceutical composition (especially: each based on the total weight of the comprimate (e.g. tablet core), and
wherein optionally the comprimate (e.g. tablet core) further comprises at least one of the following excipients:
   diluent: 10-90wt.%, more preferably 20-80wt.%, most preferably 20-60wt.%;
   binder: 1-50wt.%, more preferably 1-40wt.%, most preferably 1-30wt.%;
   disintegrant: 1-40wt.%, more preferably 1-30wt.%, most preferably 1-25wt.%;
   lubricant: 1-20wt.%, more preferably 1-15wt.%, most preferably 1-10wt.%;
   glidant: 0.1-20wt.%, more preferably 0.1-15wt.%, most preferably 0.1-10wt.%;
   antitacking agent: 1-20wt.%, more preferably 1-15wt.%, most preferably 1-10wt.%;
   each based on the total weight of the pharmaceutical composition (especially: each based on the total weight of the comprimate (e.g. tablet core)), and optionally one or more further tablet core excipients, and
   a tablet coating. The term "polymer" refers to "optional polymeric material" of the present invention.

The composition of the present invention can be prepared by technological procedures, comprising wet granulation. It has been found that wet granulation provides more beneficial results than other methods such as direct compression or dry granulation, especially when a physical stabilization of agomelatine metastable polymorphic form such as polymorphic form X and/or I, in the solid dosage form is required.

Furthermore, the present inventors provide a process for preparing a pharmaceutical composition, in particular pharmaceutical composition for oral administration, comprising agomelatine, wherein said process is
(a.) a process for preparing a pharmaceutical composition comprising agomelatine, which process comprises:
   (i) preparing a granulate comprising agomelatine (and optionally one or more excipients), in particular a granulate comprising agomelatine and pH modifier and optionally polymer (optionally one or more further excipients), preferably by wet granulation;
   (ii) optionally combining (and optionally mixing) the granulate with at least one excipient to obtain an compression mixture;
   (iii) optionally compressing the compression mixture to obtain a comprimate, in particular tablet; and
   (iv) optionally applying a film coating on the comprimate to obtain coated comprimate, in particular film coated tablet.
   or
(b.) a process for preparing a pharmaceutical composition comprising agomelatine, which process comprises:
   (i') preparing a granulate comprising at least one excipient (and optionally agomelatine), preferably by wet granulation;
   (ii') combining (and optionally mixing) the granulate with a mixture comprising agomelatine and at least one excipient to obtain a compression mixture;
   (iii') compressing the compression mixture to obtain a comprimate, in particular tablet; and
   (iv') optionally applying a film coating on the comprimate to obtain coated comprimate, in particular film coated tablet.

In the process for preparing a pharmaceutical composition, weight ratio between pH modifier and agomelatine (pH modifier: agomelatine) in the pharmaceutical composition obtainable (or prepared) by the process can be from 1:10 to 10:1, preferably 1:5 to 5:1, more preferably 1:2 to 2:1, especially 1:1.6 to 1.6:1, most preferably about 1:1.6; in particular, by adding during the process (especially during steps (i) to (iv) or steps (i') to (iv')) a total amount of pH modifier and a total amount of agomelatine in a weight ratio (pH modifier: agomelatine) which can be from 1:10 to 10:1, preferably 1:5 to 5:1, more preferably 1:2 to 2:1, especially 1:1.6 to 1.6:1, most preferably about 1:1.6.

In the process for preparing a pharmaceutical composition, weight ratio between polymer and agomelatine (polymer: agomelatine) in the pharmaceutical composition obtainable (or prepared) by the process can be from 1:10 to 10:1, preferably 1:5 to 5:1, more preferably 1:5 to 1:1, most preferably 1:1 to 1:3; in particular, by adding during the process (especially during steps (i) to (iv) or steps (i') to (iv')) a total amount of polymer and a total amount of agomelatine in a weight ratio (polymer: agomelatine) which can be from 1:10 to 10:1, preferably 1:5 to 5:1, more preferably 1:5 to 2:1, most preferably 1:3 to 1.5:1.

In the process for preparing a pharmaceutical composition, weight ratio between polymer and pH modifier (polymer: pH modifier) in the pharmaceutical composition obtainable (or prepared) by the process can be from 1:10 to 10:1, preferably 1:5 to 5:1, more preferably 1:3 to 3:1, most preferably 2:1 to 1:2; in particular, by adding during the process (especially during steps (i) to (iv) or steps (i') to (iv')) a total amount of polymer and a total amount of pH modifier in a weight ratio (polymer: pH modifier) which can be from 1:10 to 10:1, preferably 1:5 to 5:1, more preferably 1:4 to 4:1, most preferably 1:3 to 3:1.

In the process for preparing a pharmaceutical composition, weight ratio between polymer, pH modifier and agomelatine (polymer: pH modifier: agomelatine) in the pharmaceutical composition obtainable (or prepared) by the process can be 0.1-5 : 0.1-5 : 1, preferably 0.1-3 : 0.1-3 : 1, more preferably 0.1-2 : 0.1-2 : 1, most preferably 0.3-1: 0.5-1 : 1; in particular, by adding during the process (especially during steps (i) to (iv) or steps (i') to (iv')) a total amount of polymer, a total amount of pH modifier, and a total amount of agomelatine in a weight ratio which can be 0.1-5 : 0.1-5 : 1, preferably 0.1-3 : 0.1-3 : 1, more preferably 0.1-2 : 0.1-2 : 1, most preferably 0.3-1.5 : 0.5-1 : 1.

Prior to preparing the granulate, the process can comprise sieving the excipient(s) and active agent(s) for preparing the granulate. In particular prior to step (i)), the process can optionally comprise a step of sieving the agomelatine, and the optional further components. In particular prior to step (ii)), the process can optionally comprise a step of sieving the at least one excipient and the optionally present agomelatine).

In particular, pH modifier can be incorporated in the pharmaceutical composition in the granulation step (in particular in step (i) supra) or can be added as an excipient to the granulate to obtain a compression mixture (in particular in step (ii) supra) or can be both be incorporated in the pharmaceutical composition in the granulation step (in particular in step (i) supra) and added as an excipient to the granulate to obtain a compression mixture (in particular in step (ii) supra.

In the process for preparing a pharmaceutical composition (a.), step (i) can be or comprise: preparing a granulate comprising agomelatine, pH modifier, polymer, and optionally one or more further excipients.

Step (i) of preparing a granulate comprising agomelatine can be or comprise: subjecting a mixture comprising agomelatine and at least one of or both of polymer and pH modifier to granulation, preferably wet granulation, to obtain a granulate (which comprises at least one of or both of polymer and pH modifier). Preferably, the granulation liquid can comprise water and at least one of or both of polymer and pH modifier.

In one embodiment, step (i) of preparing a granulate comprising agomelatine can be or comprise: subjecting a mixture comprising agomelatine and polymer (and optionally pH modifier) to wet granulation with granulation liquid comprising pH modifier and optionally polymer to obtain a granulate comprising agomelatine, pH modifier and polymer.
In one embodiment, step (i) of preparing a granulate comprising agomelatine can be or comprise: subjecting a mixture comprising agomelatine and pH modifier (and optionally polymer) to wet granulation with granulation liquid comprising polymer and optionally pH modifier to obtain a granulate comprising agomelatine, pH modifier and polymer.
In one embodiment, step (i) of preparing a granulate comprising agomelatine can be or comprise: subjecting a mixture comprising agomelatine and pH modifier to wet granulation with granulation liquid comprising pH modifier, optionally the granulation liquid comprising polymer or being free of polymer, to obtain a granulate comprising agomelatine, and pH modifier (and optionally polymer).

In one embodiment, step (i) of preparing a granulate is carried out at inlet air temperature below 80 °C, especially below 60 °C, further especially at inlet air temperature of 25°C or below.
In one embodiment, in particular when preparing a composition comprising agomelatine Form I, process steps (i) to (iii), in particular process steps (i) to (iv), can be carried out at a temperature 25°C or below. In one embodiment, in particular when preparing a composition comprising agomelatine Form I, process steps (ii') to (iii'), in particular process steps (i') to (iv'), can be carried out at a temperature 25°C or below.

In a process for preparing a pharmaceutical composition, especially in one of processes (a.) and (b.), the granulate can be prepared by granulation which is or comprises wet granulation. In a process for preparing a pharmaceutical composition, wherein granulation is or comprises wet granulation (in particular wherein granulation in step (i) or step (i') is or comprises wet granulation), in particular wet granulation can be wet granulation with granulation liquid comprising water; preferably, the granulation liquid can further comprise at least one or both of pH modifier and polymer, especially the granulation liquid can further comprise pH modifier and optionally polymer.

In particular, step (i) can be: (i) preparing a granulate comprising agomelatine and pH modifier and optionally polymer, said granulate comprising pH modifier and agomelatine (pH modifier: agomelatine) in a weight ratio which can be from 1:10 to 10:1, preferably 1:5 to 5:1, more preferably 1:2 to 2:1, especially 1:1.6 to 1.6:1, most preferably about 1:1.6.

Furthermore, step (i) can be: (i) preparing comprising agomelatine and pH modifier and optionally polymer, said granulate comprising polymer and agomelatine (polymer: agomelatine) in a weight ratio which can be from 1:10 to 10:1, preferably 1:5 to 5:1, more preferably 1:5 to 2:1, most preferably 1:3 to 1.5:1.

Furthermore, step (i) can be: (i) preparing comprising agomelatine and pH modifier and optionally polymer, said granulate comprising polymer and pH modifier (polymer: pH modifier) in a weight ratio which can be from 1:10 to 10:1, preferably 1:5 to 5:1, more preferably 1:4 to 4:1, most preferably 1:3 to 3:1.

Furthermore, step (i) can be: (i) preparing comprising agomelatine and pH modifier and optionally polymer, said granulate comprising polymer, pH modifier and agomelatine (polymer: pH modifier: agomelatine) in a weight ratio which can be 0.1-5 : 0.1-5 : 1, preferably 0.1-3 : 0.1-3 : 1, more preferably 0.1-2 : 0.1-2 : 1, most preferably 0.3-1.5 : 0.5-1 : 1.

In particular, a process for preparing a pharmaceutical composition comprising agomelatine and pH modifier in a weight ratio of pH modifier: agomelatine from 1:10 to 10:1, preferably 1:5 to 5:1, more preferably 1:2 to 2:1, especially 1:1.6 to 1.6:1, most preferably about 1:1.6, can comprise:
(i) preparing a granulate comprising agomelatine, said preparing comprising: subjecting a mixture comprising agomelatine and optionally pH-modifier and optionally polymer to wet granulation with granulation liquid;
(ii) combining (and optionally mixing) the granulate with at least one excipient to obtain a compression mixture, said compression mixture comprising pH modifier and agomelatine in weight ratio of pH modifier: agomelatine from 1:10 to 10:1, preferably 1:5 to 5:1, more preferably 1:2 to 2:1, especially 1:1.6 to 1.6:1, most preferably about 1:1.6, wherein at least one of, two of or all of: (aa) the mixture subjected to granulation, (bb) the granulation liquid, and (cc) at least one excipient which is combined with the granulate comprise pH modifier;
(iii) compressing the compression mixture to obtain a comprimate, in particular tablet; and (iv) optionally applying a film coating on the comprimate.

In particular steps (ii') can be: (ii') combining (and optionally mixing) the granulate with a mixture comprising agomelatine and at least one excipient to obtain a compression mixture, said compression mixture comprising agomelatine and pH modifier in a weight ratio of pH modifier: agomelatine from 1:10 to 10:1, preferably 1:5 to 5:1, more preferably 1:2 to 2:1, especially 1:1.6 to 1.6:1, most preferably about 1:1.6; in said compression mixture at least one of or both of granulate and mixture comprising agomelatine can comprise pH modifier.

In a pharmaceutical composition (e.g. granulate, comprimate, in particular tablet) comprising agomelatine and pH modifier, the weight ratio of pH modifier: agomelatine can be from 1:10 to 10:1, preferably 1:5 to 5:1, more preferably 1:2 to 2:1, especially 1:1.6 to 1.6:1, most preferably about 1:1.6.

In particular, a process for preparing a pharmaceutical composition comprising agomelatine, pH modifier and optionally polymer, in a weight ratio of:
- pH modifier: agomelatine from 1:10 to 10:1, preferably 1:5 to 5:1, more preferably 1:2 to 2:1, especially 1:1.6 to 1.6:1, most preferably about 1:1.6,
- polymer: agomelatine from 1:10 to 10:1, preferably 1:5 to 5:1, more preferably 1:5 to 2:1, most preferably 1:3 to 1.5:1,
- polymer: pH modifier from from 1:10 to 10:1, preferably 1:5 to 5:1, more preferably 1:4 to 4:1, most preferably 1:3 to 3:1,
- polymer: pH modifier: agomelatine from 0.1-5 : 0.1-5 : 1, preferably 0.1-3 : 0.1-3 : 1, more preferably 0.1-2 : 0.1-2 : 1, most preferably 0.3-1.5 : 0.5-1:1,
   can comprise:
   (i) preparing a granulate comprising agomelatine, said preparing comprising: subjecting a mixture comprising agomelatine and optionally pH modifier and optionally polymer to wet granulation with granulation liquid;
   (ii) combining (and optionally mixing) the granulate with at least one excipient to obtain a compression mixture, said compression mixture comprising a weight ratio of
      - pH modifier: agomelatine from 1:10 to 10:1, preferably 1:5 to 5:1, more preferably 1:2 to 2:1, especially 1:1.6 to 1.6:1, most preferably about 1:1.6,
      - polymer: agomelatine from 1:10 to 10:1, preferably 1:5 to 5:1, more preferably 1:5 to 2:1, most preferably 1:3 to 1.5:1,
      - polymer: pH modifier from from 1:10 to 10:1, preferably 1:5 to 5:1, more preferably 1:4 to 4:1, most preferably 1:3 to 3:1,
      - polymer: pH modifier: agomelatine from 0.1-5 : 0.1-5 : 1, preferably 0.1-3 : 0.1-3 : 1, more preferably 0.1-2 : 0.1-2 : 1, most preferably 0.3-1.5 : 0.5-1:1,
   wherein at least one of, two of or all of: (aa) the mixture subjected to granulation, (bb) the granulation liquid, and (cc) at least one excipient which is combined with the granulate comprise pH modifier and polymer; (iii) compressing the compression mixture to obtain a comprimate, in particular tablet; and (iv) optionally applying a film coating on the comprimate.

In particular steps (ii') can be: (ii') combining (and optionally mixing) the granulate with a mixture comprising agomelatine and at least one excipient to obtain a compression mixture, said compression mixture comprising agomelatine, pH modifier and polymer in a weight ratio of:
- pH modifier: agomelatine from 1:10 to 10:1, preferably 1:5 to 5:1, more preferably 1:2 to 2:1, especially 1:1.6 to 1.6:1, most preferably about 1:1.6,
- polymer : agomelatine from 1:10 to 10:1, preferably 1:5 to 5:1, more preferably 1:5 to 2:1, most preferably 1:3 to 1.5:1,
- polymer: pH modifier from 1:10 to 10:1, preferably 1:5 to 5:1, more preferably 1:4 to 4:1, most preferably 1:3 to 3:1,
- polymer: pH modifier: agomelatine from 0.1-5 : 0.1-5 : 1, preferably 0.1-3 : 0.1-3 : 1, more preferably 0.1-2 : 0.1-2 : 1, most preferably 0.3-1.5 : 0.5-1:1;
in said compression mixture at least one of or both of granulate and mixture comprising agomelatine can comprise pH modifier and polymer.

In a pharmaceutical composition (e.g. granulate, comprimate, in particular tablet) comprising agomelatine, pH modifier and polymer, the weight ratio of
- pH modifier: agomelatine from 1:10 to 10:1, preferably 1:5 to 5:1, more preferably 1:2 to 2:1, especially 1:1.6 to 1.6:1, most preferably about 1:1.6,
- polymer: agomelatine from 1:10 to 10:1, preferably 1:5 to 5:1, more preferably 1:5 to 2:1, most preferably 1:3 to 1.5:1,
- polymer: pH modifier from from 1:10 to 10:1, preferably 1:5 to 5:1, more preferably 1:4 to 4:1, most preferably 1:3 to 3:1,
- polymer: pH modifier: agomelatine from 0.1-5 : 0.1-5 : 1, preferably 0.1-3 : 0.1-3 : 1, more preferably 0.1-2 : 0.1-2 : 1, most preferably 0.3-1.5 : 0.5-1 : 1.

In particular, polymer can be incorporated in the pharmaceutical composition in the granulation step (in particular step (i) supra) or can be added as an excipient to the granulate to obtain a compression mixture (in particular in step (ii) supra) or both.
Further it has been observed that processes involving wet granulation are particularly advantageous for preparing compositions comprising agomelatine. Processes involving wet granulation can contribute to avoid or minimize undesired polymorphic form transformations, and can provide even more advantageous results than e.g. hot melt processes such as hot melt extrusion or hot melt granulation.

Wet granulation can be performed by using granulation liquids, based on water. Preferably wet granulation is performed in the absence of organic solvents, especially in the absence of alcohols. Granulation liquid (used for wet granulation) preferably comprises water, and can be optionally free of organic solvent or can comprise not more than 10wt.%, preferably not more than 5 wt.% of organic solvent, based on the total weight of the granulation liquid. Especially, granulation liquid (used for wet granulation) can be optionally free of organic solvent (other than organic acid(s)) or can comprise not more than 10 wt.%, preferably not more than 5 wt.% of organic solvent (other than organic acid(s)), based on the total weight of the granulation liquid. In the context of the present application, an organic solvent can be in particular a compound comprising a carbon atom, which compound is liquid at a temperature of 20°C, especially at a pressure of 101325 Pa.

In particular, granulation liquid comprising water can be free of one or more selected from the group of R^{a}-OH, R^{b}-O-R^{c}, R^{d}-C(O)-O-R^{e}, hydrocarbons, and mixtures thereof, wherein R^{a}, R^{b}, R^{c}, R^{d}, R^{e}, can be each independently selected from alkyls, e.g. C1-C10 alkyls, (such as e.g. methanol, ethanol, diethyl ether, ethyl acetate, etc.), or can comprise not more than 10 wt.%, preferably not more than 5 wt.% of said one or more selected from the group of R^{a}-OH, R^{b}-O-R^{c}, R^{d}-C(O)-O-R^{e}, hydrocarbons, and mixtures thereof, based on the total weight of the granulation liquid. In particular, granulation liquid can consist of water or can comprise at least 60 wt.-%, optionally at least 70 wt.%, further optionally at least 75 wt.% of water, based on the total weight of the granulation liquid.

Wet granulation with granulation liquid can comprise contacting the mixture or the compound to be granulated with granulation liquid, e.g. said contacting can be or comprise spraying of granulation liquid on the mixture or compound to be granulated.

One or more excipients selected from pH modifiers, diluents, binders, disintegrants, lubricants, glidants, antitacking agents can optionally be dissolved, suspended, emulsified or dispersed into the granulation liquid which is sprayed onto the mixture, in particular powder mixture, comprising agomelatine and at least one excipient or a mixture of excipients. In another embodiment of the present invention, excipients selected from diluents, binders, disintegrants, lubricants, glidants, antitacking agents, specific polymers and pH modifiers can optionally be dissolved, suspended, emulsified or dispersed into the granulation liquid which is sprayed onto the mixture, in particular powder mixture, comprising agomelatine and at least one excipient or a mixture of excipients. In an embodiment of the present invention, granulation can be performed using granulator(s) (especially state of the art granulator(s)) such as high shear granulator, low shear granulator, or a fluid bed granulator.

In the preparation of granulate by wet granulation agomelatine and pH modifier can be incorporated partially intragranularly and partially extragranularly, alone or in combination with at least one excipient or a mixture of excipients. In another embodiment of the present invention, in the preparation of granulate by wet granulation agomelatine, polymer and pH modifier can be incorporated partially intragranularly and partially extragranularly, alone or in combination with at least one excipient or a mixture of excipients. In an embodiment of the present invention, at least one excipient or a mixture of excipients can be used partially intragranularly and partially extragranularly.

The mixing of agomelatine and at least one excipient or a mixture of excipients may be effected in conventional devices used for mixing of powder or powders, e.g. motionless (passive) mixers, fluidized bed, diffusion, biconic diffusion, uni- or biconic, tubular, cubic, planetary, Y-, V-shaped or high-shear mixers. The same equipment may be used in the preparation of compression mixture with the prior step of a granulate preparation by a wet granulation.

For drying of the granulate, prepared by wet granulation, conventional drying devices such as a fluid-bed dryer or drying chambers can be used.

Conventional equipment can be used for applying a film coating, such as a Wurster coating system or conventional perforated or non-perforated coating pans for use in pharmaceutical industry.

In another embodiment of the present invention, the wet granulating process comprises:
- optionally sieving at least one excipient or a mixture of excipients,
- granulating an optionally sieved at least one excipient or a mixture of excipients using one or more granulating liquids based on water,
- addition of excipient or a mixture of excipients to the granulate to give compression mixture;
- compressing the compression mixture to the desired solid dosage form;
- optionally, applying a film coating,
wherein:
- agomelatine is incorporated in the step of granulation, compression mixture preparation or can be divided in any ratio to be incorporated in both steps of granulation and compression mixture preparation,
- pH modifier is incorporated in the step of granulation, compression mixture preparation or can be divided in any ratio to be incorporated in both steps of granulation and compression mixture preparation.

Any possible combination of incorporation of agomelatine and polymer can be used in the wet granulation process. The term "granulating liquid based on" (e.g. water) can have the same meaning as "granulating liquid comprising" (e.g. water). The terms "granulating liquid" and "granulation liquid" can be used interchangeably herein.

In another embodiment of the present invention, the wet granulating process comprises:
- optionally sieving at least one excipient or a mixture of excipients,
- granulating an optionally sieved at least one excipient or a mixture of excipients using granulating liquid(s), based on water, organic solvent(s) or organic/water liquid solvent(s), which contain more than 60% of water;
- addition of excipient or a mixture of excipients to the granulate to give compression mixture;
- compressing the compression mixture to the desired solid dosage form;
- optionally, applying a film coating,
wherein:
- agomelatine is incorporated in the step of granulation, compression mixture preparation or can be divided in any ratio to be incorporated in both steps of granulation and compression mixture preparation,
- polymer is incorporated in the step of granulation, compression mixture preparation or can be divided in any ratio to be incorporated in both steps of granulation and compression mixture preparation,
- pH modifier is incorporated in the step of granulation, compression mixture preparation or can be divided in any ratio to be incorporated in both steps of granulation and compression mixture preparation.
Any possible combination of incorporation of agomelatine, polymer and pH modifier can be used in the wet granulation process.

In particular, preparation processes of the present invention disclosed herein (e.g. process (a.) or (b.) disclosed herein) may be characterized in that:
- agomelatine is incorporated in the step of granulation, or in the step of compression mixture preparation, or can be divided (in any ratio) to be incorporated in both steps of granulation and compression mixture preparation,
- polymer is incorporated in the step of granulation, or in the step of compression mixture preparation, or can be divided( in any ratio) to be incorporated in both steps of granulation and compression mixture preparation,
- pH modifier is incorporated in the step of granulation, or in the step of compression mixture preparation, or can be divided (in any ratio) to be incorporated in both steps of granulation and compression mixture preparation.

It is particularly preferred that the composition according to the present invention comprises granulate (i.e. intragranular phase) and an extragranular phase. Intra- and extragranular phase together comprise the compression mixture. The compression mixture can comprise or can consist of intragranular and extragranular phase.

It is preferred that granulate is prepared by wet granulation. It is particularly preferred that the wet granulation is performed in fluid-bed granulator/fluid-bed dryer.

Furthermore, it is preferred that agomelatine is comprised in intragranular phase.

Furthermore, it is preferred, that pH modifier is comprised in intragranular phase. In another embodiment of the present invention, it is preferred that pH modifier is incorporated in intragranular phase, especially by the addition by dissolving it in granulating liquid, or can be incorporated by partial or complete amount to dry mixture of powders to be granulated.

Furthermore, it is preferred, that pH modifier and polymer are comprised in intragranular phase. In another embodiment of the present invention, it is preferred that pH modifier and/or polymer are incorporated in intragranular phase, especially by complete or partial dissolving it in granulating liquid, wherein a part of pH modifier and/or polymer is incorporated by dissolving, the remaining part is incorporated by dry mixture of powders to be granulated.

In another embodiment of the present invention, it is preferred that the weight ratio between the following components of the pharmaceutical composition is:
- pH modifier: agomelatine 1:10 to 10:1, preferably 1:5 to 5:1, more preferably 1:2 to 2:1, especially 1:1.6 to 1.6:1, most preferably about 1:1.6,
- polymer: agomelatine 1:10 to 10:1, preferably 1:5 to 5:1, more preferably 1:5 to 2:1, most preferably 1:3 to 1.5:1,
- polymer: pH modifier 1:10 to 10:1, preferably 1:5 to 5:1, more preferably 1:4 to 4:1, most preferably 1:3 to 3:1,
- polymer: pH modifier: agomelatine 0.1-5 : 0.1-5 : 1, preferably 0.1-3 : 0.1-3 : 1, more preferably 0.1-2 : 0.1-2 : 1, most preferably 0.3-1.5 : 0.5-1: 1,

It is preferred that granulating liquid comprises (purified) water and polymer. In another embodiment of the present invention, it is preferred that granulating liquid comprises (purified) water, polymer and pH modifier.

In another aspect, there is provided a pharmaceutical composition comprising agomelatine, optionally a pH modifier, and optionally a polymer, which is obtainable by a process for preparing a pharmaceutical composition disclosed herein ( e.g. obtainable by a process for preparing a pharmaceutical composition, which is or comprises process (a.) or (b.) disclosed supra).

### METHODS

### DSC analysis:

Samples of agomelatine form X are characterized by differential scanning calorimetry (DSC) using a MettIerToledo DSC 1 STAR ^{e} System differential scanning calorimeter according to following method: Approximately 3 mg of a sample is accurately weighted in a 40 µl aluminium pan. The pan is tightly sealed with an aluminium lid, which is pierced before the sample is inserted into the oven. A thermogram is recorded in nitrogen atmosphere with flow rate 40 ml/min within temperature range 20 - 115 °C at heating rate 1°C/min.

The following examples illustrate the invention and are not intended to restrict the scope of the invention in any way.

### Examples

In the following table 3, compositions of the present invention are presented for 1 film coated tablet. Batch sizes of individual experiments varied from approx. 6.700 to approx. 70.000 film coated tablets. It was observed that batch size has no influence on the performance of the individual experiment.

**Table 3**

| Phase | Name of ingredient | Reference Example 1 | Reference Example 2 | Reference Example 3 | Reference Example 4 | Reference Example 5 | Reference Example 6 | Example 1 | Example 2 |
|---|---|---|---|---|---|---|---|---|---|
| Intragranular | Agomelatine | 25.00 | 25.00 | 25.00 | 25.00 | 25.00 | 25.00 | 25.00 | 25.00 |
| | Lactose monohydrate | 61.84 | 61.84 | 61.84 | 61.84 | 52.74 | 52.74 | 46.42 | 37.32 |
| | Maize starch | 26.00 | 26.00 | 26.00 | 26.00 | 26.00 | 26.00 | 26.00 | 26.00 |
| | Povidon K30 | 9.10 | 9.10 | - | - | - | - | - | - |
| | HPMC 15 mPa·s (dry mixture) | - | - | 9.10 | 4.60 | 13.70 | 9.20 | 4.60 | 9.20 |
| | HPMC 15 mPa·s (dissolved) | - | - | - | 4.50 | 4.50 | 9.00 | 4.50 | 9.00 |
| | Sodium starch glycolate | 3.90 | - | - | - | - | - | - | - |
| | Stearic acid | 2.60 | 2.60 | - | - | - | - | - | - |
| | Tartaric acid (dissolved) | - | - | - | - | - | - | 15.42 | 15.42 |
| | Tartaric acid (dry mixture) | - | - | - | - | - | - | - | - |
| | Purified water** | q.s. | q.s. | q.s. | q.s. | q.s. | q.s. | q.s. | q.s. |
| Extragranular | Sodium starch glycolate | - | 3.90 | 3.90 | 3.90 | 3.90 | 3.90 | 3.90 | 3.90 |
| | Colloidal silica | 0.26 | 0.26 | 0.26 | 0.26 | 0.26 | 0.26 | 0.26 | 0.26 |
| | Stearic acid | - | - | 2.60 | 2.60 | 2.60 | 2.60 | 2.60 | 2.60 |
| | Magnesium stearate | 1.30 | 1.30 | 1.30 | 1.30 | 1.30 | 1.30 | 1.30 | 1.30 |
| Film coating | Ready-to-use mixture* | 4.50 | 4.50 | 4.50 | 4.50 | 4.50 | 4.50 | 4.50 | 4.50 |
| | Purified water** | q.s. | q.s. | q.s. | q.s. | q.s. | q.s. | q.s. | q.s. |

| | | | | | | | | | |
|---|---|---|---|---|---|---|---|---|---|
| - not included in composition * Ready-to-use mixture comprise: hydroxypropylmethylcellulose, glycerol, macrogol, magnesium stearate, titanium dioxide, yellow iron oxide. ** Evaporated during drying (used as diluent for granulating liquid and film coating suspension, subsequently). | | | | | | | | | |

"HPMC 15 mPa·s" is HPMC having a viscosity of 15 mPa·s in a 2 weight percent aqueous solution at 20°C (especially at 20°C, and a pressure of 101325 Pa).

The respective solid components shown in the table were homogenized. The homogenized mixture was granulated in:
- high shear mixer/granulator (Reference Examples 1-3)
- fluid-bed granulator (Reference Examples 4- 6, Examples 1-2)

Granulating liquid, used for granulation, was:
- purified water (Reference Examples 1-3)
- aqueous dispersion of HPMC 15 mPa·s (Reference Examples 4-6)
- aqueous dispersion of HPMC 15 mPa·s and tartaric acid (Examples 1-2)

Irrespective to the granulation, the granulates were dried in fluid-bed dryer, e.g. the granulated mixture prepared in high shear granulator was transferred to fluid bed dryer, while in fluid-bed granulation, the drying process is going on in the same equipment.

Granulates were dried at the chamber of fluid bed dryer (Glatt GPCG 3 and Aeromatic 3/2/4 - dependent on batch size) at inlet air 30-60°C until final temperature of the granulate 30-40°C. Granulate was sieved, using rotational mill (Comil) with sieves with openings 0.8-1.4 mm.

Compression mixtures were prepared either manually or in uniconic mixer (dependent on batch size).

Tablet cores were tabletted on automatic rotary compressing machine, fitted with oval punches with dimensions 8.5 x 5.5 mm to the target weight 130 mg. Calculated maximal allowed main force during tabletting for the punches used at tabletting was 23.0 kN.

Cores were coated with film coating suspension in conventional perforated coating pan until the target weight of 134.5 mg.

The process was monitored by standard in-process controls for individual manufacturing stage, i.e. granulate, compression mixture, cores and film coated tablets, along with values of main pressure during tabletting.
Loss on drying was performed using halogen moisture analyser at 105°C for 5 minutes.
Individual weights and hardness was measured on the same 20 cores. Hardness was measured according to monograph 2.9.8. of European Pharmacopoeia 9.2 .
Disintegration time was performed in purified water at 37°C on 6 cores according to monograph 2.9.1. of European Pharmacopoeia 9.2.

**Table 4**

| Manufacturing stage | In-process control | Reference Example 1 | Reference Example 2 | Reference Example 3 | Reference Example 4 | Reference Example 5 | Reference Example 6 | Example 1 | Example 2 |
|---|---|---|---|---|---|---|---|---|---|
| Granulate | Loss on drying | 1.55°/ | 1.29°/ | 1.01% | 1.18% | 1.37% | 1.15% | 1.08% | 1.33% |
| Compression mixture | Loss on drying | 1.70% | 1.66% | 1.21% | / | / | / | 1.28% | 1.54% |
| Cores | Individual weights | 128.2-133.9 mg | 126.2-133.8 mg | 128.1-133.9 mg | 128.5-134.0 mg | 129.5-133.3 mg | 124.4-136.4 mg | 129.3-132.9 mg | 125.5-133.4 mg |
| | Hardness | 75-91 N | 68-90 N | 72-97 N | 72-96 N | 80-94 N | 75-108 N | 84-103 N | 87-118 N |
| | Disintegration time | 7-7.5 min | 7.5 min | 10-13 min | 7-9 min | 20-22 min | >45 min | 14-15 min | 32-36 min |
| | Main force | 12.5 kN | 12.4 kN | 8.6 kN | 5.8 kN | 4.0 kN | 5.9 kN | 2.9 kN | 2.7 kN |
| Film coated tablets | Average weight | 133.4 mg | 133.7 mg | 135.6 mg | 134.6 mg | 133.8 mg | 134.1 mg | 135.1 mg | 135.5 mg |
| | Assay of agomelatin in film coated tablets | 100.9% | 101.8% | 103.1% | 97.7% | 99.9% | 100.8% | 100.4% | 100.8% |

| | | | | | | | | | |
|---|---|---|---|---|---|---|---|---|---|
| / not performed | | | | | | | | | |

Based on the results of in-process control and measured parameters, i.e. main force during tabletting, it could be concluded that at lower main pressure we achieved higher hardness of cores at formulations of fluid bed granulation in comparison to high shear granulation. However, it was observed that incorporation of HPMC 15 mPa·s in the formulation prolongs disintegration time in comparison to formulation with the same quantity of povidone K30.
Furthermore, by increasing the amount of polymer (HPMC 15 mPa·s) in the formulation, significantly prolonged disintegration time of cores was observed (Examples 1 and 2). However, besides its quantity, it is also important how HPMC 15 mPa·s is incorporated in the formulation, divided by dissolving it in granulating liquid (4.50 mg and 9.00 mg) and by addition thereof in dry mixture of powders for granulate manufacturing (13.20 mg and 9.20 mg), which has effect on disintegration time of the manufactured cores, i.e. 20-22 minutes and >45 minutes, subsequently (Reference Examples 5 and 6).
The presence or absence of pH modifier (tartaric acid) in the formulation has significant influence on the tabletting process; incorporation of pH modifier (tartaric acid) significantly improves compressibility of compression mixture, which means that significantly lower main force during tabletting can be used for achieving even higher hardness, while disintegration time does not significantly changed (Reference Example 4 and Example 1).
The way of incorporation of tartaric acid in the granulate (by dissolving it in granulation liquid or addition to dry mixture of powders for granulate manufacturing) has no significant influence on disintegration time of cores.

Furthermore, an Example 3 (according to the present invention) has been carried out. This Example corresponds to Example 1, except that the tartaric acid has been not added in dissolved form, but has been added to the dry mixture. An analysis of the film-coated tablets obtained after preparation shows that the tablets according to Example 3 contain agomelatine in polymorph Form X.

Pharmaceutical composition comprising agomelatine (polymorphic form X), pH modifier (tartaric acid as pH modifier) and polymer (HPMC as polymer) as obtained according to Examples the present invention is surprisingly and unexpectedly stable in terms of chemical and physical stability. We have surprisingly found out that the polymorphic form X does not convert to other polymorphic form when subjected to stress stability conditions at 50°C / 75%RH (relative humidity) 7 days (17 days) in an open or closed container. During our study of polymorphic conversion we have found that a correlation between testing condition "50C/75% RH 7 days, closed«, and testing condition »40C/75% RH, 6 months, closed« . Short term stability results at 50°C correlates with stability results at 40°C, 6 months, which is confirmed by stability results for Ref. Ex 1 and 2.

**Table 5**

| Stability testing condition | Ref. Example 1 | Ref. Example 2 | Ref. Example 3 | Ref. Example 4 | Ref. Example 5 | Ref. Example 6 | Example 1 | Example 2 |
|---|---|---|---|---|---|---|---|---|
| t₀ | Form X | Form X | Form X | Form X | Form X | Form X | Form X | Form X |
| 50°C/75% RH 7 days | | | form II + ∼25% form X | form II + ∼30% form X | ∼70% form II + ∼30% form X | form X + 30-40% form II | Form X | FormX |
| 50°C/75% RH 17 days closed | form X + ∼10% form II | form X + ∼10-15% form II | / | / | / | / | Form X | Form X |
| 40°C/75% RH 6 months closed | Form X+ 10% form II | Form X+ 10-15% form II | | | | | | |

As evident from the table a particularly advantageous polymorphic stability is achieved in cases when the pharmaceutical compositions comprise pH modifier (tartaric acid) and polymer (HPMC). Stabilisation using polymer only was not satisfactory.

## Claims

1. Pharmaceutical composition, in particular pharmaceutical composition for oral administration, comprising
agomelatine, in particular agomelatine in crystalline form,
a pH modifier, and
optionally a polymer.

2. Pharmaceutical composition according to claim 1, wherein the pH modifier is a pH modifier which, when 1 g pH modifier is mixed with 100 g of water at 20 °C provides a pH below 5, and preferably below 3.

3. Pharmaceutical composition according to any one of the preceding claims, wherein the pH modifier is a pH modifier having a solubility in water at 20°C of more than 1 milligram of pH modifier per liter of water, preferably of more than 10 milligram of pH modifier per liter of water.

4. Pharmaceutical composition according to any one of the preceding claims, wherein the pH modifier is selected from the group consisting of acids, and mixtures thereof,
wherein optionally the pH modifier is selected from the group consisting of acetic acid, tartaric acid, and mixtures thereof.

5. Pharmaceutical composition according to any one of the preceding claims, wherein the agomelatine in crystalline form is or comprises one of agomelatine polymorphic form X, agomelatine polymorphic form I, and mixtures thereof.

6. Pharmaceutical composition according to any one of the preceding claims, wherein the polymer is or comprises polymer selected from the group consisting of cellulose ethers, and mixtures thereof,
wherein optionally the polymer is or comprises polymer selected from the group consisting of hydroxyalkyl-alkylcelluloses, and mixtures thereof, or
wherein optionally the polymer is or comprises polymer selected from the group consisting of hydroxypropyl methylcellulose (HPMC), methylcellulose (MC), ethylcellulose (EC), hydroxyethyl cellulose (HEC), hydroxypropyl cellulose (HPC), hydroxypropyl ethylcellulose (HPEC), ethylhydroxyethylcellulose (EHEC), hydroxyethyl methylcellulose (HEMC), and mixtures thereof,
wherein further optionally the polymer is or comprises hydroxypropyl methylcellulose.

7. Pharmaceutical composition according to any one of the preceding claims, which pharmaceutical composition comprises:
agomelatine in an amount of 5-50wt.%, preferably 10-40wt.%, more preferably 10-30wt.%;
pH modifier in an amount of 1-40wt.%, preferably 1-30wt.%, more preferably 1-25wt.%, especially 3-25wt.%, further especially 5-16wt.%;
optionally polymer in an amount of 1-50wt.%, preferably 1-40wt.%, more preferably 1-30wt.%, especially 4-25wt.%, each based on the total weight of the pharmaceutical composition, and optionally one or more further excipients;
wherein optionally the pharmaceutical composition is a pharmaceutical composition comprising:
agomelatine: 5-50wt.%, more preferably 10-40wt.%, most preferably 10-30wt.%;
diluent: 10-90wt.%, more preferably 20-80wt.%, most preferably 20-60wt.%;
binder: 1-50wt.%, more preferably 1-40wt.%, most preferably 1-30wt.%;
disintegrant: 1-40wt.%, more preferably 1-30wt.%, most preferably 1-25wt.%;
lubricant: 1-20wt.%, more preferably 1-15wt.%, most preferably 1-10wt.%;
glidant: 0.1-20wt.%, more preferably 0.1-15wt.%, most preferably 0.1-10wt.%;
antitacking agent: 1-20wt.%, more preferably 1-15wt.%, most preferably 1-10wt.%;
polymer: 1-50wt.%, more preferably 1-40wt.%, most preferably 1-30wt.%, especially 3-25wt.%;
pH modifier: 1-40wt.%, more preferably 1-30wt.%, most preferably 1-25wt.%, especially 3-25wt.%, further especially 5-16wt.%, each based on the total weight of the pharmaceutical composition.

8. Pharmaceutical composition according to any one of the preceding claims, which pharmaceutical composition comprises or is granulate comprising agomelatine, preferably the granulate being obtainable by wet granulation.

9. Pharmaceutical composition according to claim 8, which pharmaceutical composition comprises:
granulate comprising agomelatine, and
at least one extragranular excipient.

10. Process for preparing a pharmaceutical composition, in particular pharmaceutical composition for oral administration, comprising agomelatine, wherein said process is
(a.) a process for preparing a pharmaceutical composition comprising agomelatine, which process comprises:
(i) preparing a granulate comprising agomelatine, in particular a granulate comprising agomelatine and pH modifier and optionally polymer, preferably by wet granulation;
(ii) optionally combining the granulate with at least one excipient to obtain a compression mixture;
(iii) optionally compressing the compression mixture to obtain a comprimate, in particular tablet; and
(iv) optionally applying a film coating on the comprimate.
or
(b.) a process for preparing a pharmaceutical composition comprising agomelatine, which process comprises:
(i') preparing a granulate comprising at least one excipient, preferably by wet granulation;
(ii') combining the granulate with a mixture comprising agomelatine and at least one excipient to obtain a compression mixture;
(iii') compressing the compression mixture to obtain a comprimate, in particular tablet; and
(iv') optionally applying a film coating on the comprimate.

11. Process for preparing a pharmaceutical composition according to claim 10, wherein step (i) is or comprises:
preparing a granulate comprising agomelatine, pH modifier, polymer, and optionally one or more further excipients.

12. Process for preparing a pharmaceutical composition according to any one of claims 10 to 11, wherein step (i) of preparing a granulate comprising agomelatine is or comprises:
subjecting a mixture comprising agomelatine and at least one of or both of polymer and pH modifier to granulation, preferably wet granulation, to obtain a granulate,
or
wherein step (i) of preparing a granulate comprising agomelatine is or comprises:
subjecting a mixture comprising agomelatine and polymer (and optionally pH modifier) to wet granulation with granulation liquid comprising pH modifier and optionally polymer to obtain a granulate comprising agomelatine, pH modifier and polymer;
or
wherein step (i) of preparing a granulate comprising agomelatine is or comprises:
subjecting a mixture comprising agomelatine and pH modifier (and optionally polymer) to wet granulation with granulation liquid comprising polymer and optionally pH modifier to obtain a granulate comprising agomelatine, pH modifier and polymer;
or
wherein step (i) of preparing a granulate comprising agomelatine is or comprises:
subjecting a mixture comprising agomelatine and pH modifier to wet granulation with granulation liquid comprising pH modifier to obtain a granulate comprising agomelatine, and pH modifier,
wherein the granulation liquid optionally comprises polymer or the granulation liquid optionally is free of polymer.

13. Process for preparing a pharmaceutical composition according to any one of claims 10 to 12, wherein the granulate is prepared by wet granulation,
preferably wherein wet granulation is wet granulation with granulation liquid comprising water, optionally the granulation liquid further comprises at least one or both of pH modifier and polymer.

14. Pharmaceutical composition according to any one of claims 1 to 9, or process for preparing a pharmaceutical composition according to any one of claims 10 to 13, wherein the weight ratio of pH modifier: agomelatine is in the range from 1:10 to 10:1, preferably from 1:5 to 5:1, more preferably from 1:2 to 2:1, especially from 1:1.6 to 1.6:1, most preferably about 1:1.6.

15. Pharmaceutical composition according to any one of claims 1 to 9, which is obtainable by a process for preparing a pharmaceutical composition according to any one of claims 10 to 14.
